# EUROPEAN PATENT SPECIFICATION

(11) **EP 4 153 777 B1**
(45) Date of publication and mention of the grant of the patent: **11.02.2026**
(21) Application number: 21808376.4
(22) Date of filing: 18.05.2021
(51) Int. Cl.: C12Q 1/6806, C12N 15/10

(54) **NUCLEIC ACID SAMPLE ENRICHMENT AND SCREENING METHODS**
VERFAHREN ZUR ANREICHERUNG UND ZUM SCREENING VON NUKLEINSÄUREPROBEN
ENRICHISSEMENT D'ÉCHANTILLON D'ACIDES NUCLÉIQUES ET PROCÉDÉS DE DÉPISTAGE

(30) Priority: 18.05.2020 US 202062704616 P; 13.11.2020 US 202063113730 P
(43) Date of publication of application: 29.03.2023
(73) Proprietor: Myriad Women's Health, Inc., South San Francisco, CA 94080 (US)
(72) Inventor: CHU, Clement, South San Francisco, California 94080 (US); THEILMANN, Mark, South San Francisco, California 94080 (US); WELKER, Noah, South San Francisco, California 94080 (US); GRAUMAN, Peter, South San Francisco, California 94080 (US)
(74) Representative: Hoffmann Eitle
(86) International application number: PCT/US2021/033018
(87) International publication number: WO 2021/236680

(56) References cited:
- US-A1- 2019 106 737
- US-A1- 2019 144 848
- US-A1- 2019 287 649
- HE Q Z ET AL: "A method for improving the accuracy of non-invasive prenatal screening by cell-free foetal DNA size selection", BRITISH JOURNAL OF BIOMEDICAL SCIENCE, ROYAL SOCIETY OF MEDICINE SERVICES, LONDON, GB, vol. 75, no. 3, 30 June 2018 (2018-06-30), pages 133 - 138, XP009521937, ISSN: 0967-4845, DOI: 10.1080/09674845.2018.1468152
- PING HU: "An enrichment method to increase cell-free fetal DNA fraction and significantly reduce false negatives and test failures for non-invasive prenatal screening: a feasibility study", vol. 17, no. 1, 11 April 2019 (2019-04-11), XP093164774, ISSN: 1479-5876, Retrieved from the Internet <URL:http://link.springer.com/article/10.1186/s12967-019-1871-x/fulltext.html> DOI: 10.1186/s12967-019-1871-x
- JURAJ GAZDARICA ET AL: "Combination of Fetal Fraction Estimators Based on Fragment Lengths and Fragment Counts in Non-Invasive Prenatal Testing", INTERNATIONAL JOURNAL OF MOLECULAR SCIENCES, vol. 20, no. 16, 14 August 2019 (2019-08-14), pages 3959, XP055726743, DOI: 10.3390/ijms20163959
- MAGDALENA GRUNT: "Clinical relevance of size selection of circulating DNA", TRANSLATIONAL CANCER RESEARCH, vol. 7, no. S2, 1 March 2018 (2018-03-01), pages S171 - S184, XP093163523, ISSN: 2218-676X, DOI: 10.21037/tcr.2017.10.10
- HARRIS J K; SAHL J W; CASTOE T A; WAGNER B D; POLLOCK D D; SPEAR J R: "Comparison of Normalization Methods for Construction of Large, Multiplex Amplicon Pools for Next-Generation Sequencing", APPLIED AND ENVIRONMENTAL MICROBIOLOGY, vol. 76, no. 12, 15 June 2010 (2010-06-15), pages 3863 - 3868, XP055003922, ISSN: 0099-2240, DOI: 10.1128/AEM.02585-09
- JIANG JIE, CHEN XUELI, SUN LIYA, QING YING, YANG XUHAN, HU XIAOWEN, YANG CHAO, XU TIANLE, WANG JIJUN, WANG PENG, HE LIN, DONG CHAO: "A nalysis of the concentrations and size distributions of cell -free DNA in schizophrenia using fluorescence correlation spectroscopy", TRANSLATIONAL PSYCHIATRY, vol. 8, 104, 1 December 2018 (2018-12-01), pages 1 - 8, XP055875015, DOI: 10.1038/s41398-018-0153-3
- ANDREW G HADD; HOUGHTON JEFF; CHOUDHARY ASHISH; SAH SACHIN; CHEN LIANGJING; MARKO ADAM C; SANFORD TIFFANY; BUDDAVARAPU KALYAN; KRO: "Targeted, High-Depth, Next-Generation Sequencing of Cancer Genes in Formalin- Fixed, Paraffin-Embedded and Fine-Needle Aspiration Tumor Specimens", THE JOURNAL OF MOLECULAR DIAGNOSTICS, vol. 15, no. 2, 1 March 2013 (2013-03-01), pages 234 - 247, XP055286254, ISSN: 1525-1578, DOI: 10.1016/j.jmoldx.2012.11.006

## Description

### CROSS REFERENCE TO RELATED APPLICATIONS

This application claims priority benefit to U.S. Provisional Application No. 62/704,616 filed on May 18, 2020, entitled Pooled Nucleic Acid Sample Enrichment and Screening Methods and U.S. Provisional Application No. 63/113,730 filed November 13, 2020, also entitled Pooled Nucleic Acid Sample Enrichment and Screening Methods.

### FIELD OF THE INVENTION

The present invention relates to methods for enriching test samples for target nucleic acid molecules for further genetic screening.

### BACKGROUND

Genetic variation is known to cause medical conditions, including but not limited to hemophilia, thalassemia, Duchenne Muscular Dystrophy (DMD), Huntington's Disease (HD), Alzheimer's Disease and Cystic Fibrosis (CF). Frequently the cause of the variance is a simple addition, substitution, or deletion of a single nucleotide base in a critical gene. Additionally, birth defects are caused by chromosomal abnormalities, such as Trisomy 21 (Down's Syndrome), Trisomy 13 (Patau Syndrome), Trisomy 18 (Edward's Syndrome), Monosomy X (Turner's Syndrome) and certain sex chromosome aneuploidies such as Klinefelter's Syndrome (XXY). Another genetic variation is fetal gender, which can often be determined based on sex chromosomes X and Y. Some genetic variations may predispose an individual to any number of diseases such as, for example, diabetes, arteriosclerosis, obesity, various autoimmune diseases and cancer (e.g., colorectal, breast, ovarian, lung). Identifying genetic variances can lead to diagnosis of, or determining predisposition to, a medical condition and inform medical decisions. Research and development efforts to discover genetic abnormalities that produce adverse health consequences have identified specific genes and/or critical diagnostic markers for genetic based diseases.

For example, cell-free DNA (cfDNA) has been described and developed to serve as an effective biomarker for diseases and birth defects. For example, the discovery of cfDNA in the plasma of pregnant mothers has led to the development of several non-invasive prenatal diagnostic (NIPS) techniques in the past decade. However, detection of fetal aneuploidy and autosomal recessive disorders with cfDNA has its challenges because only a small portion of the cfDNA in maternal plasma is derived from the fetus. A primary driver of NIPS sensitivity for aneuploidy in a given maternal plasma sample is the fetal fraction (FF). Hui, L. et al., Prenat Diagn. 2020; 40:155-163. For most samples, FF values are between 4% and 30%. Wang, E. et al., Prenat Diagn. 2013; 33:662-666. Many laboratories fail samples with FF < 4% to diminish the risk of issuing false negative reports. Because the molecular and bioinformatic implementations of NIPS have evolved, diversified, and generally improved over time, sensitivity at progressively lower FF levels is platform- and laboratory dependent. Hui, L. et al., Prenat Diagn. 2020; 40:155-163; Artieri, C.G. et al., Prenat Diagn. 2017; 37:482-490. Indeed, a recently published clinical experience study demonstrated that a customized whole-genome sequencing (WGS)-based NIPS, which does not fail low-FF samples, can have comparable accuracy at high FF and low FF for the common aneuploidies on chromosomes 13, 18, and 21. Hancock, S. et al., Ultrasound Obstet Gynecol. 2020; 56:422-430. Though the common aneuploidies have long been the main focus of NIPS because of their frequency and highly penetrant phenotype, clinically actionable chromosomal anomalies span a range of sizes and can occur anywhere in the genome. Wapner, R.J. et al., Am J Obstet Gynecol. 2015; 212:332; Advani, H.V. et al., Prenat Diagn. 2017; 37:1067-1075; Pertile, M.D. et al., Sci Transl Med. 2017; 9:eaan1240. As such, a key frontier in NIPS development is to increase the resolution (i.e., detect smaller anomalies) and the scope (i.e., the number of regions) of the screen.

cfDNA is a mixture of DNA which varies in properties (*e*.*g*. size, sequence, abundance) as well as tissue of origin (*e*.*g*., maternal vs. fetal). For example, cfDNA obtained from pregnant women contains DNA of both maternal and fetal origin, cfDNA obtained from cancer patients contains DNA of both tumor and normal cellular origin, and cfDNA obtained from transplant patients contains DNA of both host and graft origin. There are many known cases in which properties of cfDNA (*e*.*g*. size) are also statistically associated with tissue of origin. For example, in cfDNA from pregnant women, fetal DNA has a smaller fragment size distribution of than maternal DNA. Fan, H. C., et al., (2010). Analysis of the Size Distributions of Fetal and Maternal Cell-Free DNA by Paired-End Sequencing. Clinical Chemistry. 56(8): 1279-1286.

cfDNA has potential use as a diagnostic biomarker for other illnesses, such as schizophrenia and cancer. In cancer patients, tumor DNA has a smaller size distribution of fragment sizes than DNA from normal tissues. Moreover, it has been shown that the cfDNA in schizophrenia patients was composed of shorter DNA molecules and showed an apoptosis-like distribution pattern. Jang et al., Translational Psychiatry 2018; 8:104.

For assays involving nucleic acid molecules with distinguishable properties that may serve as medical condition or genetic abnormality biomarkers, such as fragment size (*e*.*g*., cfDNA), a major factor influencing test performance is the relative proportion of the target DNA fraction (*e*.*g*., fetal, tumor, graft) in a sample. In the context of ctDNA, this is often referred to as the "fetal fraction or FF or cffDNA" or "tumor fraction or cftDNA." For example, the fetal fraction is the ratio of fetal DNA (*i.e.,* the proportion of cfDNA fragments that originate from the placenta) to all DNA in a cfDNA sample. The higher the target DNA fraction, the easier it is to detect target DNA properties, such as chromosomal abnormalities. Nucleic acid methylation signature is another distinguishing characteristic that may be used. For example, methylation signature differences between mothers and fetuses have been observed. Hong-Dan, W., et al., Mol. Med. Rep. 2017;15(6): 3989-3998. Moreover, an association between promoter hypermethylation and deactivation of genes involved in DNA repair resulting in certain cancer types has been shown. Jin, B. et al., Adv. Exp. Med. Biol. 2013; 754: 3-29.

It has been shown that target DNA fractions with smaller fragment size distributions may be increased by enriching the mixture for smaller fragments using electrophoresis. Liang, B., et al., Scientific Reports. 2018; 8:17675. In the process, larger fragments are discarded. The result is often a change in the proportion of target DNA in the mixture, but a loss of total mass. Target DNA fraction enrichments of 2x or greater are possible. For example, a cfDNA mixture that was previously 10% fetal DNA becomes 20% fetal DNA after enrichment for smaller DNA fragments by electrophoresis. Such enrichment processes, in this case via electrophoresis, is often referred to as "size selection."

Due to the diagnostic utility of the fetal fraction, reports and professional society statements expressing concern about low-FF samples are common. Artieri, C.G. et al., Prenat Diagn. 2017; 37:482-490; Gregg, A.R. et al., Genet Med. 2016; 18:1056-1065; Committee on Practice Bulletins - Obstetrics, Committee on Genetics, Society for Maternal-Fetal Medicine. Practice bulletin no. 163., Obstet Gynecol. 2016; 127:e123-e137. Many publications have explored and debated the merits of different approaches to handling low-FF samples: optimizing NIPS algorithms to issue confident results at low FF (Hancock, S. et al., Ultrasound Obstet Gynecol. 2020; 56:422-430), failing such samples entirely, or pursuing mitigation strategies for failed low-FF samples, such as sequential redraw (Benn, P. et al., Obstet Gynecol. 2018;132:428-435; Hunkapiller, N. et al., Fetal Diagn Ther. 2016; 40:219-223) and FF-based risk scores (McKanna, T. et al., Ultrasound Obstet Gynecol. 2019; 53:73-79; Benn, P. et al., J Genet Couns. 2019; 29:800-806). However, consensus has remained elusive. As such, the FX protocol represents an advance in NIPS because samples that would have had low FF on standard NIPS are molecularly transformed into samples that have high FF. Accompanying the increase in test performance that FX protocol affords, assay improvement will increase the confidence that providers and patients have in their results with NIPS.

Though FF may seem an immutable and intrinsic feature of a cfDNA sample, it can be altered, and strategies for increasing FF are revealed by factors that correlate with FF. Hui, L. *et al.,* 2020; 40:155-163; Peng, X.L. et al., Int J Mol Sci. 2017; 18:453; Hestand, M.S. et al., Eur J Hum Genet. 2019; 27:198-202. For instance, FF is known to increase with gestational age (Wang, E. et al., Prenat Diagn. 2013; 33:662-666) so drawing blood later in pregnancy leads to higher FF, though the effect is minor with FF increasing by <1% per week. Livergood, M.C. et al., Am J Obstet Gynecol. 2017; 216:413 e411-413 e419; Yared, E. et al., Am J Obstet Gynecol. 2016; 215:370 e1-376 e6. FF also negatively correlates with first trimester body mass index (BMI) and maternal age (Suzumori, N. et al., J Hum Genet. 2016; 61: 647-652) but these values are effectively constant for any given pregnancy. At the molecular level, it has been observed that fetal-derived cfDNA fragments tend to be shorter (Qiao, L., et al., Am J Obstet Gynecol. 2019; 221:345 e1-345 e11; Liang, B., et al., Sci Rep. 2018; 8:17675), hypermethylated (Sun, K. et al., Proc Natl Acad Sci USA. 2015; 112: E5503-E5512; Nygren, A.O., et al., Clin Chem. 2010; 56:1627-1635; Lun, F.M., et al., Clin Chem. 2013; 59:1583-1594) and enriched at different locations than maternal cfDNA fragments. Chan, K.C., et al., Proc Natl Acad Sci USA. 2016; 113: E8159-E8168. Leveraging these biases at the molecular and bioinformatic levels has the potential to multiplicatively boost the FF of every sample. US 2019/287649 A1 discloses the diagnostic sequencing of pooled cfDNA.

Moreover, conventional selection techniques (*e*.*g*., size selection) have been limited to single samples in parallel, which is inefficient and time consuming. For instance, testing 100 samples in parallel would entail performing 100 parallel electrophoresis-based size selection procedures. No methods have been developed to date that would allow for pooling samples from multiple patients prior to performing enrichment while maintaining sample origin identification. Notable technical hurdles of such pooling and enrichment techniques may be the reason why they have not been developed. For example, for cfDNA one problem with any such solution is that the relative proportion of fragments by size varies from sample to sample. Further, pooling samples in equal quantities before size selection results in unequal relative representation of the samples in the final size selected pool. Further, performing a protocol in mass, as opposed to on a sample by sample basis, reduces variation that is introduced in any sample size selection protocol. This is helpful since many sequence anomaly detection tools rely on batch level background corrections.

What is needed is procedure that can be scalably applied to samples undergoing screening processes, such as NIPS, and yields significantly higher target DNA fraction levels (e.g., FF levels) thereby increasing sensitivity and specificity for all anomalies.

### BRIEF SUMMARY OF THE INVENTION

An object of the present invention is to provide a method of enriching for and screening target nucleic acid fractions (e.g., DNA or RNA) in test samples. In another embodiment, the test samples are pooled from a plurality of test subjects.

Another object of the present invention is to provide a method of enhancing the sensitivity and resolution of genetic diagnostic assays of nucleic acid samples

In some embodiments, the nucleic acid is genomic DNA. In other embodiments, the nucleic acid is cell-free DNA, and in other embodiments the nucleic acid is FFPE DNA.

In some embodiments, the target nucleic acid fractions comprise the fetal fraction (FF) of cell-free DNA (cffDNA).

Another object of the present invention is to determine the proper volume/mass of sample specific nucleic acid libraries to use in at least one test sample such that equal amounts or concentrations of target nucleic acid fractions (*e*.*g*., within a specific size range) from each sample are represented in the at least one test sample for diagnostic assay or further selection/enrichment.

Another object of the present invention is to determine the proper volumes/masses to use in the at least one test sample by calculating a numerical offset value for each sample and then adjusting the volume/mass accordingly.

Another object of the present invention is to generate a test sample from sample specific nucleic acid libraries that is enriched for target nucleic acid fraction. In one embodiment, the test sample is enriched for nucleic acid fragments within a particular size range and contains approximately equal concentrations of nucleic acid within a specific size range from each specific sample of origin. In another embodiment, the test sample enriched for a target nucleic acid fraction also maintains sample of origin identification. In one embodiment, the enriched test sample comprises the fetal fraction (FF) of cell-free DNA. In yet another embodiment, the enriched test sample comprises hypermethylated nucleic acid sequence fragments.

In one embodiment, nucleic acid libraries are prepared/amplified for each sample of origin collected. In this embodiment, each nucleic acid library corresponds to a sample of origin in that the nucleic acid contained within a specific library was obtained from a specific sample. In another embodiment, unique markers (e.g., labels, tags) can be used to label the nucleic acid fragments in the libraries to preserve sample of origin identification. For example, sequence-based barcodes can be added to the nucleic acid fragments in each sample on one or both ends that uniquely identify the sample of origin. In another embodiment, the labeled (*e*.*g*. barcoded) nucleic acid mixtures contained in the sample specific libraries are mixed 1:1 by volume (µl) or mass (ng) ratio to produce a first test sample. In another embodiment, the distribution of nucleic acid fragments bearing characteristics of interest (e.g., DNA fragment size distribution) of the original samples is determined using known methodologies followed by a sample specific calculation of the relative quantity of nucleic acid present, e.g., the relative abundance of nucleic acid present bearing the characteristic of interest. In another embodiment, using the sample specific relative quantities, a numerical offset is calculated using the ratio of actual nucleic acid concentration in the library/predicted nucleic acid concentration in the library. In yet another embodiment, the numerical offset value is used to calculate the weighted volume or weighted nucleic acid concentration from the sample specific libraries to be added to a second test sample for further selection or enrichment of fragments bearing the characteristic of interest. In another embodiment, weighted volumes of sample specific libraries are mixed to create a second test sample where there are equal amounts of nucleic acid bearing the characteristic of interest for every sample specific library. In another embodiment, selection (or enrichment) is performed on the second test sample using conventional techniques to produce a third test sample containing nucleic acid fragments bearing the characteristic of interest. Fragments that do not have the characteristic of interest may be discarded. In one embodiment, the third test sample is sequenced using known sequencing techniques and using the sample specific labels (e.g., barcodes), sample specific nucleic acid is isolated and screened for anomalies.

In another embodiment, nucleic acid libraries are prepared/amplified for each sample of origin collected. In another embodiment, unique markers (e.g., labels, tags) can be used to label the nucleic acid fragments in the libraries to preserve sample of origin identification. For example, sequence-based barcodes can be added to the nucleic acid fragments in each sample on one or both ends that uniquely identify the sample of origin. In one embodiment, the labeled (*e*.*g*. barcoded) nucleic acid mixtures contained in the sample specific libraries are mixed 1:1 by volume (µl) or mass (ng) ratio to produce a first test sample. In one embodiment, selection (or enrichment) is performed on the first test sample using conventional techniques to produce a second test sample containing nucleic acid fragments bearing the characteristic of interest (e.g., "target nucleic acid population"). Fragments that do not have the characteristic of interest can be discarded. In one embodiment, the relative quantities of the target nucleic acid population for each sample of origin are determined using techniques including but not limited to sequencing. In other embodiments, relative quantities can be determined using other techniques, such as for example, quantitative PCR (qPCR), droplet digital PCR (ddPCR), or the like. In another embodiment, using the sample specific relative quantities, a numerical offset is calculated using the ratio of actual nucleic acid concentration in the library/predicted nucleic acid concentration in the library. In one embodiment, the numerical offset value is used to calculate the weighted volume or weighted nucleic acid concentration from the sample specific libraries to be added to a third test sample for further selection or enrichment of fragments bearing the characteristic of interest. In some embodiments, weighted volumes of sample specific libraries are mixed to create a third test sample where there are equal amounts of nucleic acid bearing the characteristic of interest for every sample specific library. In some embodiments, the third test sample is sequenced and screened the target nucleic acid population for genetic anomalies. In some embodiments, a second selection (enrichment) is performed on the third test sample and isolating the target nucleic acid population in suspension to form a fourth test sample enriched for said target nucleic acid population and comprising substantially equal proportions from each sample of origin. In some embodiments, the fourth test sample is sequenced and screened the target nucleic acid population for genetic anomalies.

### BRIEF DESCRIPTION OF THE DRAWINGS

Representative embodiments of the invention are disclosed in more detail with reference to the following figures.
FIG. 1 is a flow chart illustrating an embodiment of the methods described herein.
FIG. 2 is a graph illustrating how the methods described herein (FX protocol) increase fetal fraction (FF) across all BMI levels. For 2,401 patients who indicated BMI values on a test-requisition form, the fetal fraction levels measured before (circles) and after (triangles) the methods are plotted as a function of patients' BMI values (vertical axis). The top panel plots the histogram of samples with or without application of the methods.
FIG. 3 is a graph illustrating measurement of abundance of cfDNA fragments from chrY plotted for male-fetus pregnancies. FX protocol is labeled as "FFA" in this Figure.
FIG. 4 is plot illustrating the fold change difference in FF as a result of applying FX protocol as a function of the original FF without FX protocol. Dashed line indicates no change in FF and samples in the shaded region had increased FF with FX protocol.
FIG. 5A is a schematic of the change in median depth per autosome as a result of FX protocol. The extent of the deviation from background is itself a measure of FF and is indicated as FFₚₒₛᵢₜᵢᵥₑ.
FIG. 5B charts the increase in FFₚₒₛᵢₜᵢᵥₑ without FX protocol (circles) and with FX protocol (triangles) is shown for aneuploid samples with the indicated chromosome anomalies.

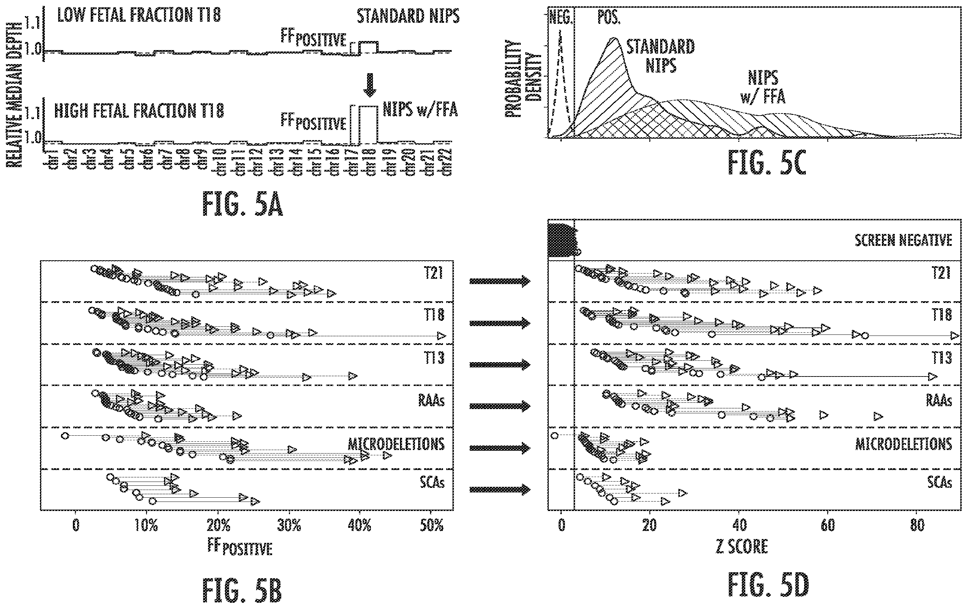

FIG. 5C charts z-scores without FX protocol ("standard NIPS") and with FX protocol ("NIPS w/FX protocol") for the same samples as in FIG. 5B stratified by their screening results and summarized either as population distributions. The distribution of screen-negative samples (NEG.; dashed line) is scaled to be of comparable height as the screen-positive distributions to the right (solid lines). The vertical solid line indicates the z-score cutoff between screen-negative (left) and screen-positive (right) results. For SCAs, only female fetus pregnancies are shown (i.e., MX and TX) because a z-score is used to identify chrX aneuploidies, whereas a two-dimensional analysis that does not use z-scores (not shown) is required for identification of XXY and XYY (FFpositive increased in all XXY and XYY pregnancies tested with FX protocol). NIPS noninvasive prenatal screening, RAA rare autosomal aneuploidies, SCA sex chromosome aneuploidies.
FIG. 5D charts z-scores without FX protocol (circles) and with FX protocol (triangles) for the same samples as in FIG. 5B stratified by their screening results and summarized as individual samples.
FIG. 6 is a plot illustrating how FX protocol improves the coefficient of variation (CV) for mapped reads versus procedures without FX protocol.

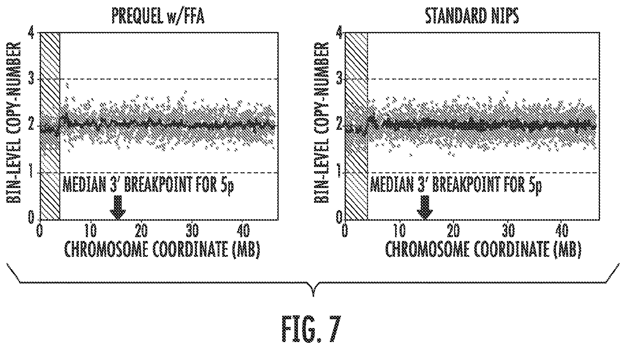

FIG. 7 is a plot comparing assay sensitivity for a short microdeletion (~3MB; shaded in blue at left of each plot) under FX protocol (labeled "FFA" in this plot) and standard NIPS conditions. The enhanced fetal fraction of FFA (left) revealed the microdeletion whereas the standard NIPS (right) screened negative. Scatter points are the bin-level normalized depth; black scatter points show the rolling median of blue points (median over 25 bin window). This particular deletion is shorter than a typical 5p deletion, for which the median 3' breakpoint is indicated (http://dbsearch.clinicalgenome.org/search/). The FF was 11% with FFA and 7% with standard NIPS.
FIG. 8 are ROC curve graphs for different classes of chromosomal abnormalities showing that FX protocol (labeled "FFA" in this plot) enables near-perfect analytical sensitivity with near-perfect analytical specificity. The sensitivity of common aneuploidies is higher with FX protocol, as is the aggregate sensitivity of RAAs.

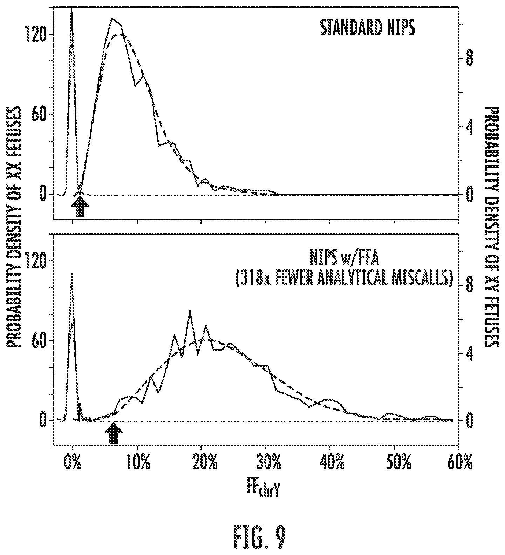

FIG. 9 is a chart showing the distribution of FFchrY values for samples called as female or male. For both standard NIPS (top) and Prequel with FX protocol (bottom), solid lines indicate raw data, and the dashed lines show best-fit traces for the female (Gaussian) and male (beta) populations. Only euploid samples are included. The arrow depicts one sample tested on both platforms, called female in standard NIPS and male with FX protocol (the fetus was confirmed to be male). After minimizing the number of estimated miscalls on each platform analytical miscalls are predicted to drop 318-fold with FX protocol.
FIG. 10 is a schematic illustrating that fetal-derived cfDNA is both less abundant and systematically shorter than maternal-derived cfDNA. Standard NIPS approaches sequence a sampling of all cfDNA, irrespective of length. Using agarose gel electrophoresis to select shorter cfDNA fragments, FX protocol (or "FFA" as it is labeled in this Figure) increases FF because relatively more of the fetal-derived cfDNA distribution is retained as compared to the maternal-derived cfDNA distribution. FX protocol increases the relative concentration of fetal-derived cfDNA fragments via size selection.

### DETAILED DESCRIPTION

While various embodiments of the invention have been shown and described herein, it will be obvious to those of skill in the art that such embodiments are provided by way of example only. Numerous variations, changes, and substitutions may occur to those skilled in the art without departing from the invention. Alternatives to the embodiments of the invention described herein may be employed. Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

Unless a term is expressly defined in this patent using the sentence "[a]s used herein, the term '_', generally refers to ... " or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning. Finally, unless a claim element is defined by reciting the word "means" and a function without the recital of any structure, it is not intended that the scope of any claim element be interpreted based on the application of 35 U.S.C. § 112, sixth paragraph.

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and nucleic acid chemistry and hybridization described below are those well-known and commonly employed in the art. The techniques and procedures are generally performed according to conventional methods in the art and various general references (see generally, Sambrook et al. MOLECULAR CLONING: A LABORATORY MANUAL, 2d ed. (1989) Cold Spring Harbor Laboratory Press, Cold Spring Harbor, N.Y.), which are provided throughout this document. The nomenclature used herein and the laboratory procedures in analytical chemistry, and organic synthetic described below are those well-known and commonly employed in the art.

As used herein, the singular forms "a," "an," and "the" include the plural reference unless the context clearly dictates otherwise.

As used herein, the terms "about" or "approximately," generally refer to within an acceptable error range for a value as determined by those skilled in the art, which will depend in part on how the value is measured or determined, i.e., the limitations of the measurement system. For example, "about" can mean within 1 or more than 1 standard deviation, per the practice in the relevant field. Alternatively, "about" can mean a range of up to 20%, up to 10%, up to 5%, or up to 1% of a given value.

As used herein, the term "subject", generally refers to an animal, such as a mammal (*e.g.,* human) or avian (*e.g.,* bird), or other organism, such as a plant. For example, the subject can be a vertebrate, a mammal, a rodent (*e*.*g*., a mouse), a primate, a simian or a human. Animals may include, but are not limited to, farm animals, sport animals, and pets. A subject can be a healthy or asymptomatic individual, an individual that has or is suspected of having a disease (*e.g.,* cancer) or a pre - disposition to the disease, and/or an individual that is in need of therapy or suspected of needing therapy. A subject can be a patient. A subject can be a microorganism or microbe (e.g., bacteria, fungi, archaea, viruses).

As used herein, the term "genome," generally refers to genomic information from a subject, which may be, for example, at least a portion or an entirety of a subject's hereditary information. A genome can be encoded either in DNA or in RNA. A genome can comprise coding regions (e.g., that code for proteins) as well as non-coding regions. A genome can include the sequence of all chromosomes together in an organism. For example, the human genome ordinarily has a total of 46 chromosomes. The sequence of all of these together may constitute a human genome.

As used herein, the terms "polynucleotide", "nucleotide", "nucleotide sequence", "nucleic acid" and "oligonucleotide" are used interchangeably and generally refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, intergenic DNA, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), small nucleolar RNA, ribozymes, cDNA, FFPE DNA, recombinant polynucleotides, branched polynucleotides, plasmids, vectors, isolated DNA of any sequence, isolated RNA of any sequence, nucleic acid probes, adapters, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component, tag, reactive moiety, or binding partner. Polynucleotide sequences, when provided, are listed in the 5' to 3' direction, unless stated otherwise.

As used herein, the term "gene" generally refers to a DNA segment that is involved in producing a polypeptide and includes regions preceding and following the coding regions as well as intervening sequences (introns) between individual coding segments (exons).

As used herein, the term "base pair" or "bp" generally refers to a partnership (*i.e*., hydrogen bonded pairing) of adenine (A) with thymine (T), or of cytosine (C) with guanine (G) in a double stranded DNA molecule. In some embodiments, a base pair may include A paired with Uracil (U), for example, in a DNA/RNA duplex.

As used herein, the term "barcode" generally refers to a known nucleic acid sequence that allows some feature of a polynucleotide with which the barcode is associated to be identified. In some embodiments, the feature of the polynucleotide to be identified is the sample from which the polynucleotide is derived. In some embodiments, barcodes are about or at least about 3, 4, 5, 6, 7, 8, 9, 10, 11, 12, 13, 14, 15, or more nucleotides in length. In some embodiments, barcodes are shorter than 10, 9, 8, 7, 6, 5, or 4 nucleotides in length. In some embodiments, barcodes associated with some polynucleotides are of different lengths than barcodes associated with other polynucleotides. In general, barcodes are of sufficient length and comprise sequences that are sufficiently different to allow the identification of samples based on barcodes with which they are associated. In some embodiments, a barcode, and the sample source with which it is associated, can be identified accurately after the mutation, insertion, or deletion of one or more nucleotides in the barcode sequence such as the mutation, insertion, or deletion of 1, 2, 3, 4, 5, 6. 7, 8, 9, 10, or more nucleotides. In some embodiments, each barcode in a plurality of barcodes differ from every other barcode in the plurality at at least three nucleotide positions, such as at least 3, 4, 5, 6, 7, 8, 9, 10, or more nucleotide positions. A plurality of barcodes may be represented in a pool of samples, each sample comprising polynucleotides comprising one or more barcodes that differ from the barcodes contained in the polynucleotides derived from the other samples in the pool. Samples of polynucleotides comprising one or more barcodes can be pooled based on the barcode sequences to which they are joined, such that all four of the nucleotide bases A, G, C, and T are approximately evenly represented at one or more positions along each barcode in the pool (such as at 1, 2, 3, 4, 5, 6, 7, 8, or more positions, or all positions of the barcode). In some embodiments, the methods of the invention further comprise identifying the sample from which a target polynucleotide is derived based on a barcode sequence to which the target polynucleotide is joined. In general, a barcode comprises a nucleic acid sequence that when joined to a target polynucleotide serves as an identifier of the sample from which the target polynucleotide was derived. In some embodiments, separate amplification reactions are carried out for separate samples using amplification primers comprising at least one different barcode sequence for each sample, such that no barcode sequence is joined to the target polynucleotides of more than one sample in a pool of two or more samples. In some embodiments, amplified polynucleotides derived from different samples and comprising different barcodes are pooled before proceeding with subsequent manipulation of the polynucleotides (such as before amplification and/or sequencing on a solid support). Pools can comprise any fraction of the total constituent amplification reactions, including whole reaction volumes. Samples can be pooled evenly or unevenly. In some embodiments, target polynucleotides are pooled based on the barcodes to which they are joined. Pools may comprise polynucleotides derived from about, less than about, or more than about 2,3, 4, 5, 6, 7, 8, 9, 10, 11, 12, 13, 14, 15, 16, 20, 25, 30, 40, 50, 75, 100, or more different samples.

As used herein, the term "sequencing," generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (*e*.*g*., single stranded DNA). Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced.

As used herein, the term "Next Generation Sequencing (NGS)" generally refers to sequencing methods that allow for massively parallel sequencing of clonally amplified and of single nucleic acid molecules during which a plurality, e.g., millions, of nucleic acid fragments from a single sample or from multiple different samples are sequenced in unison. Non-limiting examples of NGS include sequencing-by-synthesis, sequencing-by-ligation, real-time sequencing, and nanopore sequencing.

As used herein, the term "paired end sequencing" generally refers to a method based on high throughput sequencing that generates sequencing data from both ends of a nucleic acid molecule. The method generally involves sequencing from ends of a nucleic acid sequence toward the interior. Paired end sequencing is useful for determining the length of the segment of DNA that falls between two sequences.

As used herein the term "whole genome sequencing" refers to determining the complete DNA sequence of the genome at one time. As used herein, a "whole genome sequence", or WGS (also referred to in the art as a "full", "complete", or entire" genome sequence), generally refers to encompassing a substantial, but not necessarily complete, genome of a subject. In the art the term "whole genome sequence" or WGS is used to refer to a nearly complete genome of the subject, such as at least 95% complete in some usages. The term "whole genome sequence" or WGS as used herein does not encompass "sequences" employed for gene-specific techniques such as single nucleotide polymorphism (SNP) genotyping, for which typically less than 0.1% of the genome is covered. The term "whole genome sequence", or WGS as used herein does not require that the genome be aligned with any reference sequence and does not require that variants or other features be annotated.

The term "fragment size distribution" refers to any one value or a set of values that represents a length, mass, weight, or other measure of the size of molecules corresponding to a particular group (e.g. nucleic acid fragments from a particular chromosomal region). Various embodiments can use a variety of size distributions. In some embodiments, a size distribution relates to the rankings of the sizes (e.g., an average, median, or mean) of fragments of one chromosome relative to fragments of other chromosomes. In other embodiments, a size distribution can relate to a statistical value of the actual sizes of the fragments of a chromosome. In one implementation, a statistical value can include any average, mean, or median size of fragments of a chromosome. In another implementation, a statistical value can include a total length of fragments below a cutoff value, which may be divided by a total length of all fragments, or at least fragments below a larger cutoff value.

As used herein, the term "library" or "sequencing library" generally refers to a nucleic acid (*e.g.,* DNA or RNA) that is processed for sequencing, e.g., using massively parallel methods, *e.g.,* NGS. The nucleic acid may optionally be amplified to obtain a population of multiple copies of processed nucleic acid, which can be sequenced by NGS or other suitable technique.

As used herein, "fraction multiplier technology" or "FX technology" or "FX protocol" generally refers to the methods described herein to increase the yield of the target nucleic acid fraction (e.g., cffDNA) thereby increasing sensitivity for detection of anomalies, such as, for example fetal anomalies arising from copy-number changes of any size across the genome. Embodiments of methods utilizing FX protocol are described in greater detail below. In some embodiments, FX protocol leverages the reduced size of target nucleic acid molecules to increase the relative abundance of the target nucleic acid fraction. In such instances, the methods may be referred to as "fetal fraction amplification" or "FFA".

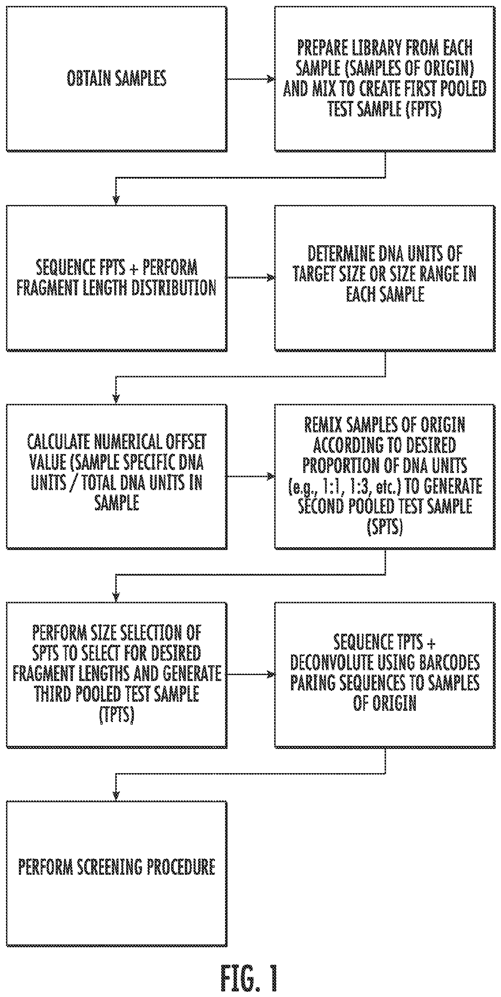

With reference to FIG. 1, in one embodiment, a biological specimen is collected from test subjects (*e*.*g*., blood plasma from pregnant females), the nucleic acid is isolated, purified, and libraries prepared and amplified using primers combined with sequence-based barcodes. In some embodiments, nucleic acid is extracted from formalin fixed paraffin embedded tissue (FFPE DNA). Formalin is commonly used as a fixative for long term tissue sample preservation and storage. While the fixation process adequately preserves the ultrastructure of the tissues, it results in various types of damage to DNA within the tissues. The DNA damage signature of FFPE DNA includes, hydrolysis of N-glycosyl bonds, deamination, oxidation, thymine dimers, nicks, and double stranded breaks. Double strand breaks result in genomic DNA fragments of varying lengths. A unique barcode sequence is used for each original sample ("sample of origin" or "original sample"). In this embodiment, unique sequence-based barcodes (also known as nucleotide sequence identifiers or NSI) or are used to label the nucleic acid fragments to preserve sample of origin identification. In general, high throughput sequencing technologies frequently involve the ligation of barcodes to nucleic acid fragments which may comprise primer binding sites used for capture, amplification and/or sequencing of the nucleic acid fragments. This technology is especially useful where samples from different origins are combined into a single high throughput sequencing run. Sequence-based barcodes allow technicians to trace back the origin of each sample from a pooled test sample. Such high throughput sequencing systems utilizing nucleotide sequence identifiers to track the origin of each sample are known in the art. A nonlimiting example of this technology is the Genome Sequencer FLX system designed by Roche which utilizes multiplexed identifier sequences (MIDs). Similar nucleotide sequence identifiers are available for other sequencing systems using next generation sequencing (NGS).

The following description involves performance of the methods on pooled samples; however, it should be noted that the processes described herein are equally applicable to a single sample and/or multiplexed samples. For example, multiplex PCR reactions can be employed to enrich for target nucleic acid. In one multiplexing embodiment, PCR primers can be designed to barcodes and a PCR reaction can be run to amplify sequences comprising the barcodes. In another embodiment, the original samples are combined to a desired mixture ratio to generate a first pooled test sample. In one embodiment, predetermined quantities of original samples are added to produce the first pooled test sample such that the units of each sample are substantially equivalent (*e.g*., 1:1:1:1, etc.) - an equal mixture ratio. "Units" can be defined as any appropriate unit of measurement, such as, for example nanograms (ng), microliters (µl), or moles (mol).

In yet another embodiment, the distribution of nucleic acid fragments bearing a specific characteristic (*e*.*g*., fragment size, molecular weight, methylation state) for the first pooled test sample is assayed. For example, in the case of cfDNA or FFPE DNA, paired-end sequencing can be used to deduce the fragment size distribution of each original sample within the first pooled test sample. Paired-end sequencing is known in the art and was originally described in Smith, M. W. et al. (1994). Genomic sequence sampling: a strategy for high resolution sequence-based physical mapping of complex genomes. Nature Genetics. 7: 40-47. Paired-end sequencing obtains information for both ends of each DNA molecule. By finding the coordinates of the 2 sequences on the genome through sequence alignment, one can deduce the length of the DNA fragment. A single sequencing experiment yields sequence and size information for millions - billions of DNA fragments. Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). It should be understood that other suitable techniques are available to determine fragment size distribution. For example, fluorescence correlation spectroscopy as described in Jiang, J., et al. (2018). Analysis of the concentrations and size distributions of cell-free DNA in schizophrenia using fluorescence correlation spectroscopy. Translational Psychiatry. 8:104.

In one embodiment, once the fragment size distribution within the first pooled test sample is determined, then the sample specific relative quantities (*i.e*., in units of choice) of DNA fragments within a target fragment size ranges (*e*.*g*., 100 to 165bp) within length bins or, alternatively, DNA fragments of specific target sizes (*e*.*g*., 165bp) may be calculated for each original sample. Sample specific relative quantities can be calculated using amplification procedures, such as polymerase chain reaction (PCR), quantitative PCR (qPCR), droplet digital PCR (ddPCR), and isothermal amplification. In another embodiment, using the sample specific relative quantities, a numerical offset value can be calculated by determining the ratio of sample specific relative quantity (units) / total units in first pooled test sample. In yet another embodiment, the numerical offset value is used to calculate the weighted number of units (e.g., µl, ng, etc.) from the sample specific libraries to be added to a second pooled test sample for fragment size selection.

In one embodiment, the weighted number of units (e.g., based on mass, volume, etc.) of each sample of origin are mixed together to generate the second pooled test sample. In some embodiments, the weighted number of units are mixed such that the units are in substantially equal proportions. In some embodiments, unequal amounts (predetermined sample specific units) of one or more samples of origin are mixed together depending on the assay being performed. Once the second pooled test sample is generated, fragment size selection can be performed to select for the desired fragment lengths. In some embodiments, gel electrophoresis can be used to isolate, excise, and purify the desired nucleic acid size fraction and generate a third pooled test sample containing nucleic acid fragments within the target size range or of specific target size. In one embodiment, nucleic acid electrophoretic separation followed by the recovery of the desired fragment lengths is used. Various known electrophoretic processes may be used for this purpose, but in one embodiment, the NIMBUS Select^{™} workstation with Ranger Technology^{™} for high throughput nucleic acid size selection may be used.

It should be understood that alternative techniques for fragment selection may be used, for example, bisulfite conversion techniques followed by on-column purification for methylated DNA; methylated DNA immunoprecipitation (based on nucleic acid methylation relative to other nucleic acid); solid support capture (*e*.*g*., affinity column), such as an antibody-coated spin column; synchronous (or non-synchronous) coefficient of drag alteration sizing (SCODA); solid phase reversible immobilization sizing (*e*.*g*., using carboxylated magnetic beads); affinity chromatography processes, or combinations of PCR amplification with varied lengths of amplicons and microchip separation.

In another embodiment, following preparation of the third pooled test sample comprising a nucleic acid mixture enriched for the target nucleic acid fragments of a specific size or size range, as well as containing the predetermined proportions (equal or variable proportions) from each of the original samples, the third pooled test sample is sequenced by next generation sequencing (NGS) or the like. Once the third pooled test sample is sequenced, barcodes may be deconvoluted via available software programs on the market to pair reads to sample of origin based on barcode sequences as described in greater detail above. Following read pairing to sample of origin, the sequencing reads can then be analyzed as desired depending on the screening being performed.

In an alternative embodiment, the numerical offset value is determined by performing fragment size selection on the first pooled sample omitting the fragment size distribution step described above via paired end sequencing, for example. In this embodiment, a predetermined amount of the first pooled test sample is used to isolate, extract, and generate a second pooled test sample containing nucleic acid fragments within the target size range or of a specific target size. In one embodiment, electrophoretic separation of nucleic acid followed by recovery of the desired lengths of nucleic acid fragments is used, as described above. In some embodiments, following recovery, the second pooled test sample is sequenced and the relative abundance of target fragments within each sample of origin may be inferred based on the number of reads assigned to a specific sample within sample specific bins. then the sample specific relative quantities (*i.e*., in units of choice) of DNA fragments within a target fragment size ranges (*e*.*g*., 100 to 165bp) within length bins or, alternatively, DNA fragments of specific target sizes (*e*.*g*., 165bp) may be calculated for each original sample. Sample specific relative quantities can be calculated using amplification procedures, such as polymerase chain reaction (PCR), quantitative PCR (qPCR), droplet digital PCR (ddPCR), and isothermal amplification. In another embodiment, using the sample specific relative quantities, a numerical offset value can be calculated by determining the ratio of sample specific relative quantity (units) / total units in first pooled test sample. In yet another embodiment, the numerical offset value is used to calculate the weighted number of units (e.g., µl, ng, etc.) from the sample specific libraries to be added to a second pooled test sample for fragment size selection.In this embodiment, a numerical offset value is determined based on the relative abundances observed from the sample specific sequencing reads. Aliquots from each sample of origin, adjusted based on the numerical offset value, are pooled to generate a third pooled test sample, and the fragment size selection/sequencing steps repeated. Ideally, equal relative abundances (based on molar concentrations, molecular weights, etc.) of the target fragments are present in the third pooled test sample, which is also enriched for the target fragments. In some embodiments, a second fragment size selection on the third pooled test sample can be performed using conventional techniques and the target nucleic acid population is isolated in suspension to form a fourth pooled test sample enriched for said target nucleic acid population and comprising substantially equal proportions from each said sample of origin. In some embodiments, the third pooled test sample and/or the fourth pooled test sample is sequenced to screen the target nucleic acid population for genetic abnormalities.

In one embodiment, FX protocol can be combined with whole-genome sequencing (WGS) based NIPS. For example, WGS-based NIPS (without FX protocol) has been configured to identify novel microdeletions anywhere in the genome, but its sensitivity and resolution is limited to microdeletions exceeding 7Mb in length. Since many microdeletions span <7Mb, increasing sensitivity for small regions across the genome could have great clinical value. The resolution limit of genome-wide copy number variant detection is driven by the relative amount of signal in a sample (dictated by the relative amount of FF in a sample and the size of the CNV) and the amount of noise present in a sample (dictated by depth to which a sample is sequenced) (e.g., it is more challenging to detect small deletions in samples with low FF). Attempts to increase resolution by deeper sequencing provides diminished returns and quickly yields and economically inviable screening test. Therefore, methods to increase FF (fetal fraction) are preferable under these circumstances; hence, the impact and applicability of FX protocol.

### EXAMPLES

For each example discussed below, all samples were from patients who had consented to deidentified research and received testing with Prequel NIPS. The study was granted an institutional review board (IRB) exemption by Advarra (Pro0042194).

### Example 1

Plasma was separated from a 10 ml whole-blood sample via centrifugation at 1600g for 10 min. using a two-step centrifugation process and plasma was transferred to microcentrifuge tubes and centrifuged at 16,000 x g for 10 min. The plasma was stored at -80 degree C before DNA extraction. DNA fragments were extracted from 0.6 ml cell-free plasma using the Circulating Nucleic Acid Kit (Qiagen, GE). An Ion Plus Fragment Library Kit (Life Technologies, USA) for the Ion Proton Platform was used to construct sequencing libraries for each plasma sample and the libraries quantified on a Qubit Fluorometer - each sample specific library containing substantially the same concentration of total DNA. Sample specific libraries are barcoded for sample of origin identification. Each library contains different amounts of DNA within a specific size range (100 to 165 bp) or a specific target size (165 bp). As shown in Table 1, samples 1-5 (remaining samples not shown) were mixed at a 1:1 ratio (10 ng each) to generate a first pooled test sample (FPTS).

In some embodiments, an alternative extraction and library preparation protocol involves extraction of the target nucleic acid fraction (e.g., cffDNA) from plasma using silanol-coated magnetic beads (Dynabeads, ThermoFisher) to yield samples at a relatively uniform concentration and fragment size or length (e.g. 165 bp). The target nucleic acid fraction was quantified (PicoGreen, ThermoFisher) and converted into a barcoded next-generation (NGS)-competent sequencing library suitable for Illumina platform using manufacturer's instructions. Libraries were amplified via 12 rounds of polymerase chain reaction (PCR) (KAPA HiFi HotStart PCR Kit, Roche) before magnetic bead-based PCR cleanup followed by another round of quantification.

Fragment size distribution of each sample within the FPTS was determined and the relative amount of DNA (ng) within the target size range (100-165bp) was obtained. A 2 µl sample from each sample represented in the FPTS was analyzed for fragment size distribution using a Fragment Analyzer (Advanced Analytical Technologies, Ames Iowa). With continued reference to Table 1, the proportion of DNA for each sample 1-5 within the target size range relative to the total units added (10ng) to the FPTS is calculated. The values obtained were used to calculate the total amounts of DNA needed from each of the five samples to have equal and predetermined DNA units (1 ng) within the target size range.

Original library samples were remixed according to the calculation at volumes that would add 1 ng of DNA within the target size range for each sample to generate second pooled test sample (SPTS). SPTS were subjected to fragment size selection procedures using 2% E-Gel EX CloneWell Agarose Gels (Invitrogen, Carlsbad, CA, USA) as in Qiao et al. and Liang et al. A piece of E-Gels contains six effective wells and each well can run a mixed sample which contains five samples of the DNA sequencing library. DNA within target size range was retrieved from the bottom wells on the gel and the selected library (i.e., third pooled test sample (TPTS) was sequenced using an Ion Proton system (Life Technologies). Other sequencing strategies may be used, for example, the Illumina HiSeq 4000 followed by processing via a custom bioinformatics pipeline.

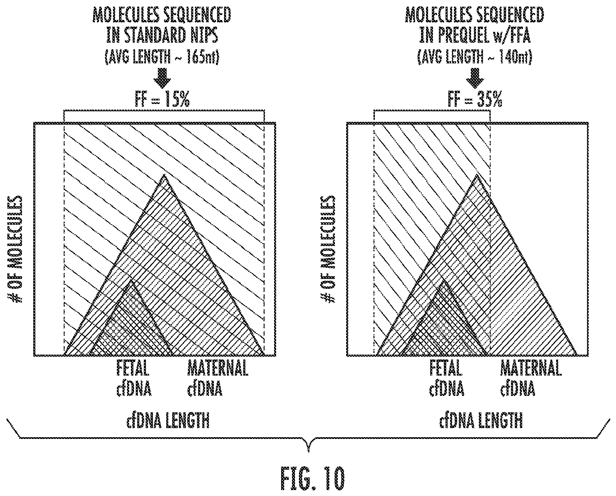

Other strategies for fragment size selection include electrophoresis on 2% agarose cassettes (BluePippin, Sage Science) following the manufacturer's instructions for "range" mode. Short fragments are eluted from the gel until the desired target size of the eluted DNA is, for example, 140 nt. See Figure 10. For fetal cfDNA, this size adequately retains fetal cfDNA and depletes maternal cfDNA. The size-selected libraries had higher FF because fetal-derived fragments comprise a higher fraction of the total size-selected cfDNA.

**Table 1**

| Sample | Total DNA units (ng) | DNA units within range (ng) | Proportion within range | Desired target units within range (ng) | Total DNA units (ng) required for sample meet desired target |
|---|---|---|---|---|---|
| 1 | 10 | 1 | 0.1 | 1 | 10 |
| 2 | 10 | 3 | 0.3 | 1 | 3.33 |
| 3 | 10 | 5 | 0.5 | 1 | 2 |
| 4 | 10 | 7 | 0.7 | 1 | 1.43 |
| 5 | 10 | 10 | 1 | 1 | 1 |

Barcodes were deconvoluted via available software programs on the market to pair reads to sample of origin based on barcode sequences and then reads were screened for relevant medical condition or chromosomal abnormality, *e.g*., fetal aneuploidy.

### Example 2

To validate FX protocol analytically, plasma was extracted from 10 ml of whole blood samples (1,264 NIPS patient samples and 66 controls tested on 11 batches) using a two-step centrifugation process and plasma was transferred to microcentrifuge tubes and centrifuged at 16,000 x g for 10 min to remove residual cells and obtain cell free plasma which was stored at -80 degree C before DNA extraction. DNA fragments were extracted from 0.6 ml cell-free plasma using the Circulating Nucleic Acid Kit (Qiagen, GE). An Ion Plus Fragment Library Kit (Life Technologies, USA) for the Ion Proton Platform was used to construct sequencing libraries for each plasma sample and the libraries quantified on a Qubit Fluorometer - each sample specific library containing substantially the same concentration of total DNA. Sample specific libraries are barcoded for sample of origin identification. Each library contains different amounts of DNA within a specific size range (100 to 165 bp) or a specific target size (165 bp). Samples were mixed at a 1:1 ratio (10 ng each) to generate a first pooled test sample (FPTS).

Each patient sample was processed through two workflows: (1) standard WGS-based NIPS without FX protocol or (2) WGS-based NIPS with FX protocol. FX protocol leverages the reduced size of fetal-derived cfDNA molecules to increase the relative abundance of fetal cfDNA. The workflows were executed completely independently, each beginning with the extraction of cfDNA from replicate plasma aliquots.

Fragment size distribution of each sample within the FPTS was determined and the relative amount of DNA (ng) within the target size range (100-165bp) was obtained. A 2 µl sample from each sample represented in the FPTS was analyzed for fragmentation size distribution using a Fragment Analyzer (Advanced Analytical Technologies, Ames Iowa). The proportion of DNA for each sample within the target size range relative to the total units added (10ng) to the FPTS was calculated. The values obtained were used to calculate the total amounts of DNA needed from each of the five samples to have equal and predetermined DNA units (1 ng) within the target size range. Original library samples were remixed according to the calculation at volumes that would add 1 ng of DNA within the target size range for each sample to generate second pooled test sample (SPTS). SPTS were subjected to fragment size selection procedures using in this case E-Gel CloneWell Agarose Gels (Invitrogen, Carlsbad, CA, USA). A piece of E-Gels contains six effective wells and each well can run a mixed sample which contains five samples of the DNA sequencing library. DNA within target size range was retrieved from the bottom wells on the gel and the selected library (i.e., third pooled test sample (TPTS) was sequenced using an Ion Proton system (Life Technologies).

### A. FX protocol increases fetal fraction (FF)

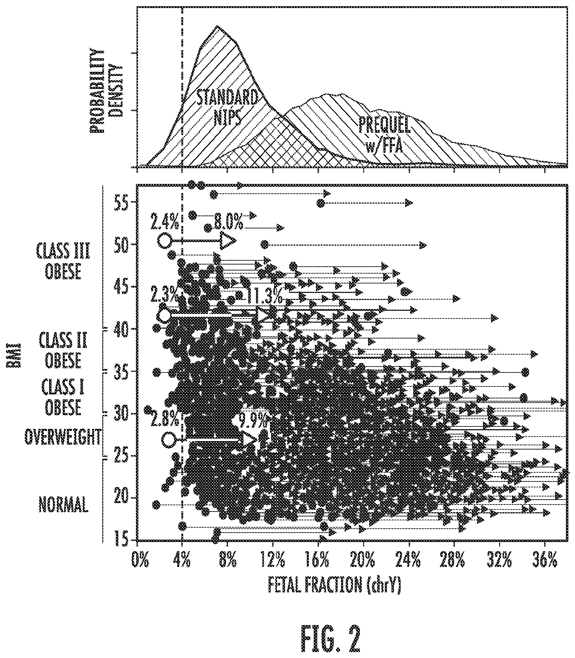

To directly measure the impact of FX protocol, samples were tested with both the standard - NIPS and FX protocol focusing particularly on the number of samples with FF > 4%. According to the American College of Medical Genetics and Genomics (ACMG), the threshold for low FF is less than 4%. As shown in FIG. 2 (top), 3.7% of samples tested with the standard NIPS protocol had an FF (e.g., fraction containing cffDNA) less than 4%. With FX protocol, however, zero samples had an FF of less than 4%. The minimum FF observed in the FX group was 4.9%. As it has been observed that samples from patients with high BMI tend to have low FF and cause elevated test failures with standard NIPS, samples were partitioned by their BMI classes (FIG. 2 (bottom)). Even at the highest BMI level (class III obesity), where 16% of the samples had low FF with standard NIPS, every sample tested with FX protocol had FF > 4% (minimum FF observed among class III patients was 7.1%).

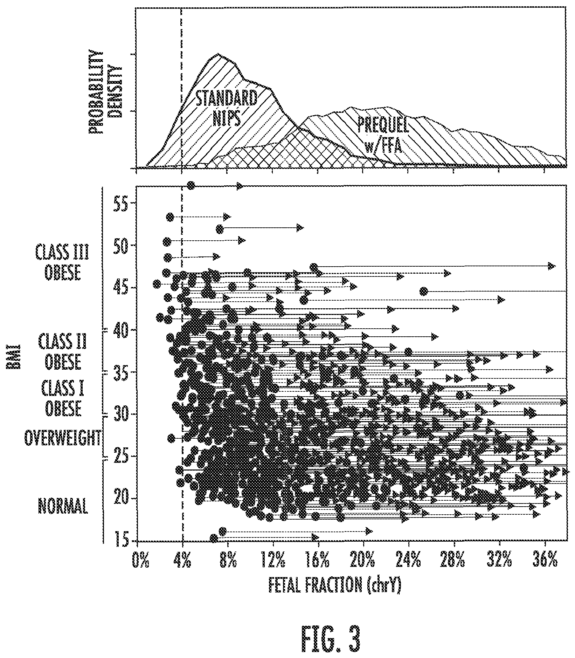

To confirm that FX protocol did not artifactually increase FF by corrupting our FF-inference regression model, we verified that the density of reads from chrY in pregnancies with male fetuses rose commensurately. *See* FIG. 3. Thus, FX protocol increases FF by directly increasing the relative abundance of fetal-derived cfDNA fragments in each sequenced sample.

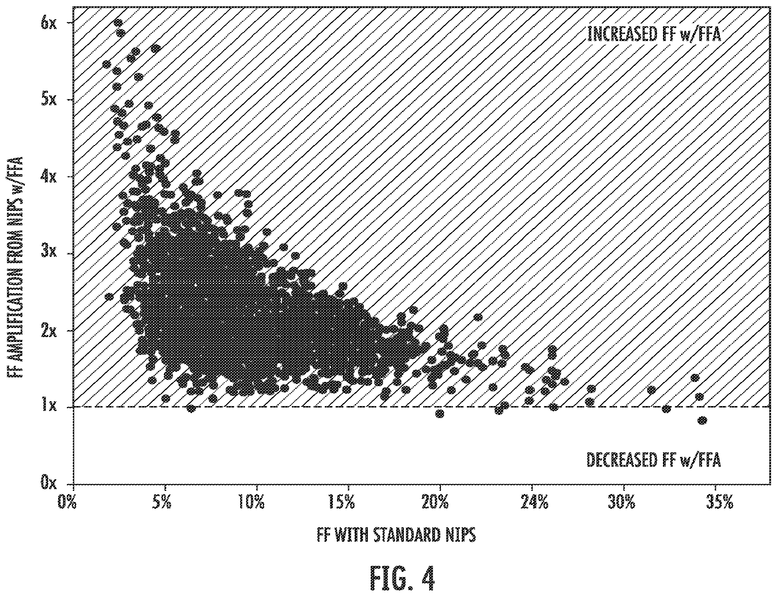

Sample-level changes in FF resulting from the FX protocol to determine whether the upward shift in the overall FF distribution may obscure downward-shifting FF in a subset of samples. *See* FIG. 3. FIG. 4 shows the relative gain in FF conferred by FX protocol. Notably, 2395 of the 2401 samples tested (99.8%) had an increase in FF with FX protocol with an average FF increase of 2.3-fold. The relative sample-level gain in FF varied as a function of FF (FIG. 3): samples that were at low FF (<4%) with standard NIPS had the largest FF gain with an average of 3.9-fold higher FF after undergoing FX protocol. Consistent with the FF gain diminishing at higher original FF levels, the six samples in which FF decreased with FX protocol had a median FF value of 27.8% (minimum 6.5%), and the FF with FX protocol remained high (median: 25.4%, minimum: 6.4%).

### B. FX protocol increases NIPS sensitivity

In the same manner that fetal fraction (FF) can be directly measured in male-fetus pregnancies from the relative NGS depth of chrX and chrY, it is possible to measure FF of aneuploid samples via the relative NGS depth on the aneuploid chromosome (FF ₚₒₛᵢₜᵢᵥₑ / FIG. 5A). FF ₚₒₛᵢₜᵢᵥₑ is directly proportional to the z-score of an aneuploid region, and a higher z-score means that aneuploidy is more likely to be detected. Therefore, if FX protocol increases FF ₚₒₛᵢₜᵢᵥₑ of aneuploidy regions, then FX protocol also increases NIPS sensitivity.

In every positive sample tested - across common aneuploidies (e.g., sex chromosome aneuploidy (SCA)), rare autosomal aneuploidies (RAA), and microdeletions, FX protocol yielded an increase in FF (FIG. 5B). An increase in FF ₚₒₛᵢₜᵢᵥₑ without FX protocol is denoted by gray circles and an increase in FF ₚₒₛᵢₜᵢᵥₑ with FX protocol is denoted by purple triangles. This upward shift in the distribution of FF was unchanged by FX. FX also increased z-scores for every tested aneuploid sample, whereas the z-score distribution for euploid samples was unchanged (FIG. 5C-D). Larger z-score separation between positive and negative samples heightens the ability to discriminate such samples and thereby lessens the changes of false negatives and false positives. Together these observations demonstrate that FX protocol directly increases the concentration of fetal-derived reads in each sample and enhances the sensitivity and specificity of NIPS.

A sample that screened negative for the 5p micro deletion with standard NIPS but positive with FX protocol (FIGS. 7, 5B, 5D) provided further support for the enhanced sensitivity for fetal chromosome abnormalities that FX protocol confers. For this 3-MB microdeletion, the copy-number change was conspicuously apparent in the FX protocol data (FIG. 7), converting a z-score below the calling threshold into one above the threshold (FIG. 3D, microdeletions track).

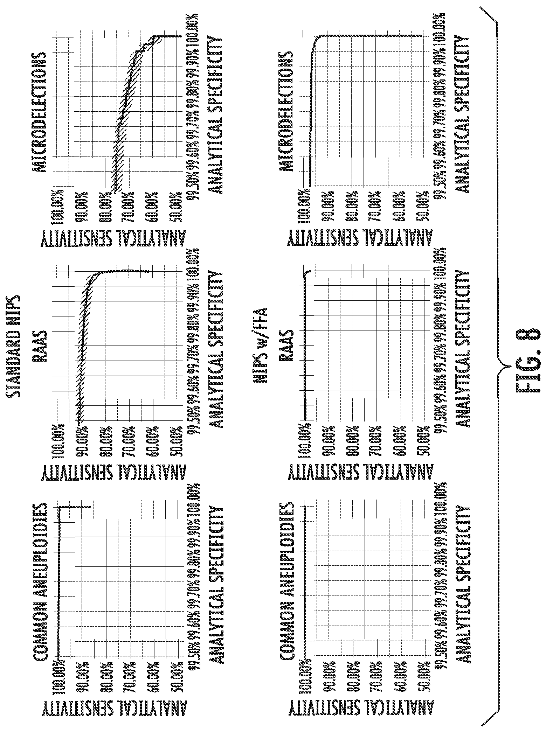

To quantify the gain in sensitivity and specificity achievable with FX protocol, we analyzed the relationship between various clinical and technical metrics, such as z-scores, depth, incidence, and FF (see Materials and methods"). The ROC curves (FIG. 8) for different classes of chromosomal abnormalities show that FX protocol enables near-perfect analytical sensitivity with near-perfect analytical specificity (Table 2). The sensitivity of common aneuploidies-shown to be high in our clinical experience without FX protocol -is higher with FX protocol, as is the aggregate sensitivity of RAAs (FIG. 8). However, the gain in microdeletion sensitivity is substantial: with FX protocol, the aggregate sensitivity for five common microdeletions is 97.2% at a joint specificity of 99.8%. For DiGeorge syndrome in particular, FX protocol has an analytical sensitivity of 95.6% with an analytical specificity of 99.95%. Table 2 (below).

**Table 2**

| | **Analytical Sensitivity** | **Analytical Specificity** |
|---|---|---|
| Common Aneuploidies (aggregate) | 99.988% ± 0.004% | 99.968% ± 0.005% |
| T21 | 99.990% ± 0.005% | 99.996% ± 0.001% |
| T18 | 99.990% ± 0.002% | 99.996% ± 0.001% |
| T13 | 99.978% ± 0.005% | 99.976% ± 0.005% |
| RAAs (aggregate) | 99.695% ± 0.305% | 99.981% ± 0.010% |
| Microdeletions (aggregate) | 97.172% ± 0.054% | 99.767% ± 0.012% |
| DiGeorge syndrome (22q11.2) | 95.633% ± 0.071% | 99.949% ± 0.005% |

In addition to assessing performance with the ROC analysis above, we also observed that all samples with a confirmed aneuploidy or microdeletion were correctly identified with FX protocol (Table 3).

### Table 3

**Table 3: Prequel with FFA had perfect concordance relative to orthogonally confirmed outcome. Sixty-seven samples had confirmed outcome via diagnostic procedure or assessment at birth. One confirmed negative sample was included in the analysis and contributes to the specificity calculation. 95% confidence intervals (CI) were calculated with the Jeffreys-interval approach.**

| **Aneuploidy** | **# confirmed positive** | **FFA clinical sensitivity** | **FFA clinical specificity** |
|---|---|---|---|
| T21 | 24 | 100% (CI: 90.2% -100%) | 100% (CI: 94.5% -100%) |
| T18 | 24 | 100% (CI: 90.2% -100%) | 100% (CI: 94.5% -100%) |
| T13 | 10 | 100% (CI: 78.3% -100%) | 100% (CI: 95.8% -100%) |
| Microdeletion | 2 | 100% (CI: 33.3% -100%) | 100% (CI: 96.3% -100%) |
| SCA | 7 | 100% (CI: 70.8% -100%) | 100% (CI: 96.0% -100%) |

Moreover, the results were repeatable and reproducible within and across batches, respectively (Tables 4 and 5). Together, these experiments establish the analytical validity of FX protocol.

### Table 4

**Table 4: Intra-run repeatability. Six screen-positive samples (two each for T13, T18, and T21) and 50 screen-negative samples were tested in duplicate on a single flow cell. The observed percent agreement was 100%.**

| | | Replicate 1 | |
|---|---|---|---|
| | | Negative | Positive |
| Replicate 2 | Negative | 50 | 0 |
| | Positive | 0 | 6 |

### Table 5

**Table 5: Inter-run reproducibility. Nine samples that were screen-positive for common aneuploidies (three each for T13, T18, and T21) and 66 screen-negative samples were tested in duplicate on separate flowcells. The observed percent agreement was 100%.**

| | | Replicate 1 | |
|---|---|---|---|
| | | Negative | Positive |
| Replicate 2 | Negative | 66 | 0 |
| | Positive | 0 | 9 |

Finally, the number of false negative (FN) results per sample screened with standard NIPS or Prequel with Fx protocol (labeled FFA here) were estimated The false negative rate (FNR) is calculated as (1 - sensitivity), where sensitivity is the analytical sensitivity estimated from the ROC analysis. The number of FN per sample screened is the product of the FNR and the prevalence. Prevalence numbers can range based on age and other factors, thus prevalence values are approximate, expressed as 1 in *x*, where *x* is rounded to nearest hundred (for common aneuploidies and RAAs) or the nearest 1000 (for common microdeletions and 22q11.2). The five common microdeletions are 1p, 4p, 5p, 15q11, and 22q11.2. The rates of FNR and FN / sample screened are much lower when FX protocol is used to enhance the fetal fraction. See Table 6 below.

**Table 6**

| | | | Standard NIPS Prequel w/ FFA | | | |
|---|---|---|---|---|---|---|
| | | Approximate Prevalence | FNR | Approximate FN / sample screened | FNR | Approximate FN / sample screened |
| Common aneuploidies | | 1 in 100 | 1 in 183 | 1 in 18,300 | 1 in 8,333 | 1 in 833,300 |
| RAAs | | 1 in 200 | 1 in 7 | 1 in 1,400 | 1 in 344 | 1 in 66,800 |
| Common microdeletions | | 1 in 1,000 | 1 in 4 | 1 in 4,000 | 1 in 36 | 1 in 36,000 |
| | DiGeorge Syndrome (22q11.2) | 1 in 2,000 | 1 in 3 | 1 in 6,000 | 1 in 22.9 | 1 in 45,800 |

### C. FX protocol increases sex-calling accuracy

Sex miscalls in NIPS arise from limitations that are either biological (e.g., true fetal mosaicism, vanishing twin) or technical (e.g., low FF). While the former poses inherent challenges (many sex miscalls occur at FF far greater than 4%), the latter can be mitigated by FX protocol due to its ability to increase the FF of all samples and thereby remove borderline calls. FIG. 9 shows distributions of FFchrY (i.e., the FF as measured from the NGS read density on chromosome Y) for male fetus and female fetus pregnancies as observed for standard NIPS and FX protocol. Notably, the separation between male and female FFchrY distributions is larger with FX protocol, reducing the chance of sex miscalls due to borderline FFchrY values by about 318×. Underscoring the improvement, one sample tested in the validation study (FIG. 9, orange arrow) was borderline in standard NIPS and miscalled as XX; however, the sample was clearly XY upon screening with FX protocol, and the pregnancy was orthogonally confirmed via ultrasound to be male.

### Example 3

A goal of many next generation sequencing (NGS) based tests is to consolidate samples prior to sequencing in equal amounts. Ideally all samples would receive the exact number of reads they require to maintain test performance. In many cases, for consistency, this number of reads would be equal across all samples and the coefficient of variation (CV) for mapped reads would be 0. However, due to errors in the process (*i.e.* liquid handling, quantification, etc.) this is often not the case. This is even further exacerbated when pooled samples are size selected to isolate only a particular size range of nucleic acid.

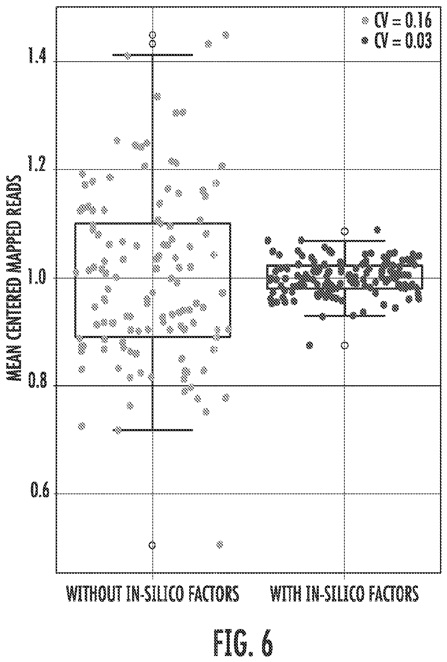

In this experiment, ~120 NGS libraries we consolidated (or pooled) in equimolar concentrations and a gel-based size selection to isolate fragments between 200-250 base pairs was performed by gel electrophoresis. The pooled NGS libraries were then sequenced on an Illumina sequencer and downstream analyses to determine the number of reads that mapped to the genome for every sample was performed. Referring to FIG. 6, the distribution of the mean centered mapped reads is indicated in the left-hand boxplot below the label "without in-silico factors." While every library was initially pooled at equimolar concentrations, since each sample had a different number of fragments within the 200-250 base pair size bin, the distribution of those reads is quite broad with the lowest read count samples receiving one third the number of reads as the highest read count samples. The CV for these samples was 0.16.

The same equimolar pool was then sequenced on a small-scale Illumina sequencing platform and paired end data was used to determine the fragment length distribution for every sample in the pool. The relative amount of DNA that was present within the 200-250bp size range was determined which was used to create "factors" that can be applied to the original quantification value, named here "in silico factors." Using these updated quantification values, the NGS libraries were re-consolidated such that each library contained an equimolar amount of DNA within the 200-250 bp size range. This re-consolidated pool was then subjected to the same 200-250 bp gel-based size selection, Illumina sequencing, and analyses as outlined above. The distribution of mean centered mapped reads is indicated in the right-hand boxplot. Since samples were now pooled based on the number of molecules present in the 200-250bp size bin, the CV of mapped reads for this consolidated pool is much lower at 0.03.

Lower CV (*i.e.* tighter distribution) of mapped reads allows for lower costs and/or fewer failed samples, since many assays that utilize NGS have a minimum read threshold required for analysis. Libraries with wide distributions of mapped reads often have samples that do not receive a sufficient number of reads. A mitigation strategy for this is combining fewer samples together in a consolidated pool, but this comes with the trade-off of increased cost. This workflow allows for size selection of a pool of DNA samples while still maintaining a tight distribution of mapped reads (low CV)."

Here we validated and characterized the performance of a NIPS that applies FX protocol to every sample. For 99.8% of samples tested, FF increased with FX protocol, with the average gain being 2.3-fold. Low-FF samples received the largest FF scaling, and of 2401 samples tested, 3.7% had low FF before FX protocol, but none had low FF after FX protocol. The gain in FF is molecular and not algorithmic: FX protocol distinguishes between maternal and fetal DNA, and it increases the relative proportion of fetal DNA in the sample undergoing WGS. Though NIPS showed high sensitivity and specificity for common aneuploidies across the FF spectrum without FX protocol technology, application of FX protocol increases performance for each type of aneuploidy. The gain was particularly substantial for microdeletions.

FX protocol has a dramatic impact on the performance of microdeletion screening in NIPS. For common microdeletions, the expected aggregate sensitivity increases (Table 1, FIG. 8), reaching 97.2% with FX protocol. In the past, when microdeletion sensitivity and specificity were low for microdeletions, the American College of Obstetricians and Gynecologists (ACOG) recommended against microdeletion screening; however, we expect that sensitivity >97% and specificity >99% for microdeletions could allow professional societies to consider the clinical merits rather than the technological limitations of screening for microdeletions.

Beyond the common microdeletions, our data suggest that FX protocol will increase the resolution of gwCNV detection, enabling confident identification of microdeletions below the current limit of 7 MB achievable with standard NIPS. Short microdeletions in samples with low FF can be challenging to detect with NIPS and limit sensitivity, but FX protocol raises the achievable sensitivity limit by reducing the frequency of low-FF samples. Notably, the 22q11.2 microdeletion, which causes DiGeorge syndrome, most commonly spans ~2-3 MB and has an expected sensitivity of 95.6% with FX protocol. To ensure that false positives are rare, the resolution limit for novel gwCNV detection may need to be above 3 MB, but dbVar contains more than a thousand unique pathogenic microdeletions between 3 MB and 7 MB in size, a number of which are associated with clinically serious phenotypes, so any gains in resolution should increase the utility of NIPS for patients and providers.

Even if two NIPS laboratories were to test the same plasma sample, the reported FF and sensitivity for aneuploidy may differ due to variations in the laboratories' respective molecular and computational protocols. For instance, based on differing methods of aligning, filtering, counting, and analyzing NGS reads, a laboratory reporting 8% FF could have higher aneuploidy sensitivity than a laboratory reporting 10% FF. These differences complicate interlab comparisons of NIPS performance, especially since laboratories demonstrate performance on different sample sets and with different study designs (e.g., clinical experience study vs. analytical validation study). As such, it can be difficult to make conclusive statements about relative NIPS performance. However, here we have demonstrated an unequivocal NIPS performance gain: two protocols (standard NIPS and FX protocol) were compared on a single set of samples within a single laboratory using a single aneuploidy-calling algorithm. FF increased 2.3-fold on average, and this FF increase resulted from a higher frequency of fetal-derived NGS reads. Beyond showing evidence for a relative gain in performance, the ROC analysis we performed yields an estimate of analytical sensitivity and specificity in an unbiased cohort reflective of a large population of clinical samples.

The FX protocol strategy described herein increases the FF of a sample at the molecular level via size selection upstream of sequencing, yet it is also possible to increase FF via algorithmic size selection downstream of sequencing. Specifically, the bioinformatics pipeline could calculate each fragment's length based on the respective mapping positions of its paired-end reads and upweight shorter fragments in the analysis. However, the disadvantage of this bioinformatic approach is that substantial resources would still be consumed by sequencing longer fragments-likely to be maternal-derived-that contribute little to fetal aneuploidy detection. By contrast, when performing molecular size selection upstream of sequencing, all of the sequenced fragments have elevated likelihood of being fetal-derived.

## Claims

1. A method of enhancing the sensitivity and resolution of genetic diagnostic assays of pooled nucleic acid samples comprising the steps of:
a. isolating and purifying nucleic acid from a plurality of test subjects to generate corresponding samples of origin to generate at least one sample of origin;
b. preparing a library for each test subject wherein the nucleic acid fragments are barcoded and wherein each library corresponds to a specific sample of origin;
c. adding a first number of nucleic acid units from each sample of origin to form a first pooled test sample;
d. determining the fragment size distribution within each sample of origin;
e. determining the abundance of a target nucleic acid population in each sample of origin;
f. calculating a unique numerical offset value for each sample of origin;
g. adding a second number of nucleic acid units from each sample of origin based on the unique numerical offset value to form a second pooled test sample; and
h. performing fragment size selection on the second pooled test sample and isolating the target nucleic acid population in suspension to form a third pooled test sample enriched for said target nucleic acid population, wherein said third pooled test sample is ready for diagnostic assay.

2. The method of claim 1, further comprising the step of sequencing said third pooled test sample and screening the target nucleic acid population for genetic anomalies; or further comprising the step of pairing the nucleic acid fragments in the third pooled test sample with the respective sample of origin; or
further comprising the step of performing whole genome sequencing.

3. The method of claim 1 wherein said fragment size distribution is determined by sequencing, optionally wherein said sequencing is paired-end sequencing;
wherein said fragment size distribution is determined by fluorescence correlation spectroscopy;
wherein said nucleic acid is genomic DNA; wherein said nucleic acid is FFPE DNA; wherein said nucleic acid is RNA;
wherein said nucleic acid is cell-free DNA; or
wherein said nucleic acid is isolated from whole blood.

4. The method of claim 1, wherein said unique numerical offset value is calculated by dividing the abundance of the target nucleic acid population determined in step e by the first number of nucleic acid units.

5. The method of claim 4, wherein said target nucleic acid population is a fetal fraction of said cell-free DNA;
wherein said target nucleic acid population is the tumor fraction of said cell-free DNA; or wherein said target nucleic acid population are fragments of nucleic acid comprising a particular methylation signature, optionally wherein said methylation signature is hypermethylation or hypomethylation.

6. The method of claim 1, wherein said target nucleic acid population is enriched for fragments within a predetermined length range;
wherein said target nucleic acid population is enriched for fragments of a predetermined length;
wherein said target nucleic acid population is enriched for fragments comprising a particular methylation signature, optionally wherein said methylation signature is hypermethylation or hypomethylation;
wherein said fragment size selection is performed using gel electrophoresis;
wherein said first and second number of nucleic acid units is selected from the group consisting of microliters, nanograms, and moles; or
wherein said pooled test sample comprises between 2 and 1000 different samples.

7. A method of enhancing the sensitivity and resolution of genetic diagnostic assays of pooled nucleic acid samples comprising the steps of:
a. isolating and purifying nucleic acid from a plurality of test subjects to generate corresponding samples of origin;
b. preparing a library for each test subject wherein the nucleic acid fragments are barcoded and wherein each library corresponds to a specific sample of origin;
c. adding a first number of nucleic acid units from each sample of origin to form a first pooled test sample;
d. performing fragment size selection on the first pooled test sample and isolating the target nucleic acid population in suspension to form a second pooled test sample enriched for said target nucleic acid population;
e. determining the abundance of a target nucleic acid population in each sample of origin;
f. calculating a unique numerical offset value for each sample of origin;
g. adding a second number of nucleic acid units from each sample of origin based on the unique numerical offset value to form a third pooled test sample enriched for said target nucleic acid population;
h. performing a second fragment size selection on the third pooled test sample and isolating the target nucleic acid population in suspension to form a fourth pooled test sample enriched for said target nucleic acid population and comprising substantially equal proportions from each said sample of origin, wherein said fourth pooled test sample is ready for diagnostic assay.

8. The method of claim 7, wherein step f is performed by sequencing;
wherein said sequencing is paired-end sequencing;
wherein step f is performed by quantitative PCR; or
wherein step f is performed by digital PCR, optionally wherein said digital PCR is droplet digital PCR.

9. The method of claim 7, further comprising the step of sequencing said fourth pooled test sample and screening the target nucleic acid population for genetic anomalies; or
further comprising the step of pairing the nucleic acid fragments in the fourth pooled test sample with the respective sample of origin.

10. The method of claim 7, wherein said nucleic acid is genomic DNA;
wherein said nucleic acid is FFPE DNA;
wherein said nucleic acid is RNA;
wherein said nucleic acid is cell-free DNA; or
wherein said nucleic acid is isolated from whole blood.

11. The method of claim 7, wherein said unique numerical offset value is calculated by dividing the abundance of the target nucleic acid population determined in step e by the first number of nucleic acid units.

12. The method of claim 7, wherein said target nucleic acid population is a fetal fraction of said cell-free DNA;
wherein said target nucleic acid population is the tumor fraction of said cell-free DNA; wherein said target nucleic acid population are fragments comprising a particular methylation signature;
wherein said methylation signature is hypermethylation or hypomethylation;
wherein said target nucleic acid population is enriched for nucleic acid fragments within a predetermined length range;
wherein said target nucleic acid population is enriched for fragments of a predetermined length;
wherein said target nucleic acid population is enriched for fragments comprising a particular methylation signature;
wherein said methylation signature is hypermethylation or hypomethylation;
wherein said fragment size selection is performed using gel electrophoresis; or
wherein said first and second number of nucleic acid units is selected from the group consisting of microliters, nanograms, and moles.

13. The method of claim 7, further comprising the step of performing whole genome sequencing.

14. The method of claim 7, wherein said pooled test sample comprises between 2 and 1000 different samples.

15. A method of enhancing the sensitivity and resolution of genetic diagnostic assays comprising the steps of:
a. isolating and purifying nucleic acid from at least one test subject to generate at least one sample of origin;
b. preparing a nucleic acid library for said at least one test subject wherein the nucleic acid fragments are barcoded and wherein said nucleic acid library corresponds to said at least one sample of origin;
c. adding a first number of nucleic acid units from said nucleic acid library to form a first test sample;
d. determining the fragment size distribution within said nucleic acid library;
e. calculating the abundance of a target nucleic acid population in said nucleic acid library;
f. calculating a unique numerical offset value for said nucleic acid library;
g. adding a second number of nucleic acid units from said nucleic acid library based on the unique numerical offset value to form a second test sample; and
h. performing fragment size selection on the second test sample and isolating the target nucleic acid population in suspension to form a third test sample enriched for said target nucleic acid population, wherein said third test sample is ready for diagnostic assay.

## Patentansprüche

1. Verfahren zum Verbessern der Sensitivität und Auflösung genetischer Diagnosetests gepoolter Nukleinsäureproben, umfassend die Schritte:
a. Isolieren und Reinigen von Nukleinsäure aus einer Vielzahl von Testsubjekten, um entsprechende Ursprungsproben zu erzeugen, um mindestens eine Ursprungsprobe zu erzeugen;
b. Erstellen einer Bibliothek für jedes Testsubjekt, wobei die Nukleinsäurefragmente mit einem Barcode versehen sind, und wobei jede Bibliothek einer spezifischen Ursprungsprobe entspricht;
c. Hinzufügen einer ersten Anzahl von Nukleinsäureeinheiten aus jeder Ursprungsprobe, um eine erste gepoolte Testprobe zu bilden;
d. Bestimmen der Fragmentgrößenverteilung innerhalb jeder Ursprungsprobe;
e. Bestimmen der Häufigkeit einer Zielnukleinsäurepopulation in jeder Ursprungsprobe;
f. Berechnen eines eindeutigen numerischen Offset-Werts für jede Ursprungsprobe;
g. Hinzufügen einer zweiten Anzahl von Nukleinsäureeinheiten aus jeder Ursprungsprobe basierend auf dem eindeutigen numerischen Offset-Wert, um eine zweite gepoolte Testprobe zu bilden; und
h. Durchführen einer Fragmentgrößenauswahl an der zweiten gepoolten Testprobe und Isolieren der Zielnukleinsäurepopulation in Suspension, um eine dritte gepoolte Testprobe zu bilden, die mit der Zielnukleinsäurepopulation angereichert ist, wobei die dritte gepoolte Testprobe für einen diagnostischen Test bereit ist.

2. Verfahren nach Anspruch 1, weiter umfassend den Schritt der Sequenzierung der dritten gepoolten Testprobe und des Screenings der Zielnukleinsäurepopulation auf genetische Anomalien; oder weiter umfassend den Schritt des Paarens der Nukleinsäurefragmente in der dritten gepoolten Testprobe mit der jeweiligen Ursprungsprobe; oder weiter umfassend den Schritt des Durchführens einer Gesamtgenomsequenzierung.

3. Verfahren nach Anspruch 1, wobei die Fragmentgrößenverteilung durch Sequenzierung bestimmt wird, optional wobei die Sequenzierung eine Paired-End-Sequenzierung ist; wobei die Fragmentgrößenverteilung durch Fluoreszenzkorrelationsspektroskopie bestimmt wird;
wobei die Nukleinsäure genomische DNA ist; wobei die Nukleinsäure FFPE-DNA ist;
wobei die Nukleinsäure RNA ist;
wobei die Nukleinsäure zellfreie DNA ist; oder
wobei die Nukleinsäure aus Vollblut isoliert wird.

4. Verfahren nach Anspruch 1, wobei der eindeutige numerische Offset-Wert berechnet wird, indem die Häufigkeit der in Schritt e bestimmten Zielnukleinsäurepopulation durch die erste Anzahl von Nukleinsäureeinheiten geteilt wird.

5. Verfahren nach Anspruch 4, wobei die Zielnukleinsäurepopulation eine fötale Fraktion der zellfreien DNA ist;
wobei die Zielnukleinsäurepopulation die Tumorfraktion der zellfreien DNA ist; oder
wobei die Zielnukleinsäurepopulation Fragmente von Nukleinsäuren sind, die eine bestimmte Methylierungssignatur umfassen, optional wobei die Methylierungssignatur eine Hypermethylierung oder Hypomethylierung ist.

6. Verfahren nach Anspruch 1, wobei die Zielnukleinsäurepopulation mit Fragmenten innerhalb eines vorbestimmten Längenbereichs angereichert ist;
wobei die Zielnukleinsäurepopulation mit Fragmenten einer vorbestimmten Länge angereichert ist;
wobei die Zielnukleinsäurepopulation mit Fragmenten angereichert ist, die eine bestimmte Methylierungssignatur umfasst, optional wobei die Methylierungssignatur Hypermethylierung oder Hypomethylierung ist;
wobei die Fragmentgrößenauswahl unter Verwendung von Gelelektrophorese durchgeführt wird;
wobei die erste und zweite Anzahl von Nukleinsäureeinheiten aus der Gruppe bestehend aus Mikrolitern, Nanogramm und Mol ausgewählt ist; oder
wobei die gepoolte Testprobe zwischen 2 und 1000 verschiedene Proben umfasst.

7. Verfahren zum Verbessern der Sensitivität und Auflösung genetischer Diagnosetests gepoolter Nukleinsäureproben, umfassend die Schritte:
a. Isolieren und Reinigen von Nukleinsäure aus einer Vielzahl von Testsubjekten, um entsprechende Ursprungsproben zu erzeugen;
b. Erstellen einer Bibliothek für jedes Testsubjekt, wobei die Nukleinsäurefragmente mit einem Barcode versehen sind, und wobei jede Bibliothek einer spezifischen Ursprungsprobe entspricht;
c. Hinzufügen einer ersten Anzahl von Nukleinsäureeinheiten aus jeder Ursprungsprobe, um eine erste gepoolte Testprobe zu bilden;
d. Durchführen einer Fragmentgrößenauswahl an der ersten gepoolten Testprobe und Isolieren der Zielnukleinsäurepopulation in Suspension, um eine zweite gepoolte Testprobe zu bilden, die mit der Zielnukleinsäurepopulation angereichert ist;
e. Bestimmen der Häufigkeit einer Zielnukleinsäurepopulation in jeder Ursprungsprobe;
f. Berechnen eines eindeutigen numerischen Offset-Werts für jede Ursprungsprobe;
g. Hinzufügen einer zweiten Anzahl von Nukleinsäureeinheiten aus jeder Ursprungsprobe basierend auf dem eindeutigen numerischen Offset-Wert, um eine dritte gepoolte Testprobe zu bilden, die mit der Zielnukleinsäurepopulation angereichert ist;
h. Durchführen einer zweiten Fragmentgrößenauswahl an der dritten gepoolten Testprobe und Isolieren der Zielnukleinsäurepopulation in Suspension, um eine vierte gepoolte Testprobe zu bilden, die mit der Zielnukleinsäurepopulation angereichert ist und im Wesentlichen gleiche Anteile aus jeder der Ursprungsproben umfasst, wobei die vierte gepoolte Testprobe für einen diagnostischen Test bereit ist.

8. Verfahren nach Anspruch 7, wobei Schritt f durch Sequenzierung durchgeführt wird;
wobei die Sequenzierung eine Paired-End-Sequenzierung ist;
wobei Schritt f durch quantitative PCR durchgeführt wird; oder
wobei Schritt f durch digitale PCR durchgeführt wird, optional wobei die digitale PCR eine digitale Droplet-PCR ist.

9. Verfahren nach Anspruch 7, weiter umfassend den Schritt der Sequenzierung der vierten gepoolten Testprobe und des Screenings der Zielnukleinsäurepopulation auf genetische Anomalien; oder
weiter umfassend den Schritt des Paarens der Nukleinsäurefragmente in der vierten gepoolten Testprobe mit der jeweiligen Ursprungsprobe.

10. Verfahren nach Anspruch 7, wobei die Nukleinsäure genomische DNA ist;
wobei die Nukleinsäure FFPE-DNA ist;
wobei die Nukleinsäure RNA ist;
wobei die Nukleinsäure zellfreie DNA ist; oder
wobei die Nukleinsäure aus Vollblut isoliert wird.

11. Verfahren nach Anspruch 7, wobei der eindeutige numerische Offset-Wert berechnet wird, indem die Häufigkeit der in Schritt e bestimmten Zielnukleinsäurepopulation durch die erste Anzahl von Nukleinsäureeinheiten geteilt wird.

12. Verfahren nach Anspruch 7, wobei die Zielnukleinsäurepopulation eine fötale Fraktion der zellfreien DNA ist;
wobei die Zielnukleinsäurepopulation die Tumorfraktion der zellfreien DNA ist;
wobei die Zielnukleinsäurepopulation Fragmente sind, die eine bestimmte Methylierungssignatur umfassen;
wobei die Methylierungssignatur Hypermethylierung oder Hypomethylierung ist;
wobei die Zielnukleinsäurepopulation mit Nukleinsäurefragmenten innerhalb eines vorbestimmten Längenbereichs angereichert ist;
wobei die Zielnukleinsäurepopulation mit Fragmenten einer vorbestimmten Länge angereichert ist;
wobei die Zielnukleinsäurepopulation mit Fragmenten angereichert ist, die eine bestimmte Methylierungssignatur umfassen;
wobei die Methylierungssignatur Hypermethylierung oder Hypomethylierung ist;
wobei die Fragmentgrößenauswahl unter Verwendung von Gelelektrophorese durchgeführt wird; oder
wobei die erste und zweite Anzahl von Nukleinsäureeinheiten aus der Gruppe bestehend aus Mikrolitern, Nanogramm und Mol ausgewählt ist.

13. Verfahren nach Anspruch 7, weiter umfassend den Schritt des Durchführens einer Gesamtgenomsequenzierung.

14. Verfahren nach Anspruch 7, wobei die gepoolte Testprobe zwischen 2 und 1000 verschiedene Proben umfasst.

15. Verfahren zum Verbessern der Sensitivität und Auflösung genetischer Diagnosetests, umfassend die Schritte:
a. Isolieren und Reinigen von Nukleinsäure von mindestens einem Testsubjekt, um mindestens eine Ursprungsprobe zu erzeugen;
b. Erstellen einer Nukleinsäurebibliothek für mindestens ein Testsubjekt, wobei die Nukleinsäurefragmente mit einem Barcode versehen sind, und wobei die Nukleinsäurebibliothek der mindestens einen Ursprungsprobe entspricht;
c. Hinzufügen einer ersten Anzahl von Nukleinsäureeinheiten aus der Nukleinsäurebibliothek, um eine erste Testprobe zu bilden;
d. Bestimmen der Fragmentgrößenverteilung innerhalb der Nukleinsäurebibliothek;
e. Berechnen der Häufigkeit einer Zielnukleinsäurepopulation in der Nukleinsäurebibliothek;
f. Berechnen eines eindeutigen numerischen Offset-Werts für die Nukleinsäurebibliothek;
g. Hinzufügen einer zweiten Anzahl von Nukleinsäureeinheiten aus der Nukleinsäurebibliothek basierend auf dem eindeutigen numerischen Offset-Wert, um eine zweite Testprobe zu bilden; und
h. Durchführen einer Fragmentgrößenauswahl an der zweiten Testprobe und Isolieren der Zielnukleinsäurepopulation in Suspension, um eine dritte Testprobe zu bilden, die mit der Zielnukleinsäurepopulation angereichert ist, wobei die dritte Testprobe für einen diagnostischen Test bereit ist.

## Revendications

1. Procédé pour améliorer la sensibilité et la résolution des tests de diagnostic génétique sur des échantillons d'acides nucléiques regroupés comprenant les étapes consistant à :
a. isoler et purifier l'acide nucléique d'une pluralité de sujets testés afin de générer des échantillons d'origine correspondants pour générer au moins un échantillon d'origine ;
b. préparer une bibliothèque pour chaque sujet testé dans laquelle les fragments d'acide nucléique sont codés par un code à barres et dans laquelle chaque bibliothèque correspond à un échantillon d'origine spécifique ;
c. ajouter un premier nombre d'unités d'acide nucléique de chaque échantillon d'origine pour former un premier échantillon de test groupé ;
d. déterminer la distribution de la taille des fragments dans chaque échantillon d'origine ;
e. déterminer l'abondance d'une population d'acides nucléiques cibles dans chaque échantillon d'origine ;
f. calculer une valeur de décalage numérique unique pour chaque échantillon d'origine ;
g. ajouter un deuxième nombre d'unités d'acide nucléique de chaque échantillon d'origine sur la base de la valeur de décalage numérique unique pour former un deuxième échantillon de test groupé ; et
h. effectuer une sélection de la taille des fragments sur le deuxième échantillon de test groupé et isoler la population d'acides nucléiques cibles en suspension pour former un troisième échantillon de test groupé enrichi pour ladite population d'acides nucléiques cibles, dans lequel ledit troisième échantillon de test groupé est prêt pour un test diagnostique.

2. Procédé selon la revendication 1, comprenant en outre l'étape consistant à séquencer ledit troisième échantillon de test groupé et à dépister la population d'acides nucléiques cibles pour y chercher des anomalies génétiques ; ou
comprenant en outre l'étape consistant à apparier les fragments d'acide nucléique dans le troisième échantillon de test groupé avec l'échantillon d'origine respectif ;
ou comprenant en outre l'étape consistant à effectuer un séquençage du génome entier.

3. Procédé selon la revendication 1 dans lequel ladite distribution de taille des fragments est déterminée par séquençage, éventuellement dans lequel ledit séquençage est un séquençage à extrémités appariées ; dans lequel ladite distribution de taille des fragments est déterminée par spectroscopie de corrélation de fluorescence ;
dans lequel ledit acide nucléique est de l'ADN génomique ; dans lequel ledit acide nucléique est de l'ADN FFPE ;
dans lequel ledit acide nucléique est de l'ARN ;
dans lequel ledit acide nucléique est de l'ADN extracellulaire ; ou
dans lequel ledit acide nucléique est isolé à partir de sang total.

4. Procédé selon la revendication 1, dans lequel ladite valeur de décalage numérique unique est calculée en divisant l'abondance de la population d'acides nucléiques cibles déterminée à l'étape e par le premier nombre d'unités d'acides nucléiques.

5. Procédé selon la revendication 4, dans lequel ladite population d'acides nucléiques cibles est la fraction fœtale dudit ADN extracellulaire ;
dans lequel ladite population d'acides nucléiques cibles est la fraction tumorale dudit ADN extracellulaire ; ou dans lequel ladite population d'acides nucléiques cibles sont des fragments d'acide nucléique comprenant une signature de méthylation particulière, éventuellement dans lequel ladite signature de méthylation est une hyperméthylation ou une hypométhylation.

6. Procédé selon la revendication 1, dans lequel ladite population d'acides nucléiques cibles est enrichie en fragments d'une longueur comprise dans une plage prédéterminée ; dans lequel ladite population d'acides nucléiques cibles est enrichie en fragments d'une longueur prédéterminée ;
dans lequel ladite population d'acides nucléiques cibles est enrichie en fragments comprenant une signature de méthylation particulière, éventuellement dans lequel ladite signature de méthylation est une hyperméthylation ou une hypométhylation ;
dans laquelle ladite sélection de la taille des fragments est effectuée par électrophorèse sur gel ;
dans laquelle lesdits premier et deuxième nombres d'unités d'acide nucléique sont sélectionnés dans le groupe constitué par les microlitres, les nanogrammes et les moles ; ou
dans laquelle ledit échantillon de test regroupé comprend entre 2 et 1000 échantillons différents.

7. Procédé pour améliorer la sensibilité et la résolution des tests de diagnostic génétique sur des échantillons d'acides nucléiques regroupés comprenant les étapes consistant à :
a. isoler et purifier l'acide nucléique d'une pluralité de sujets testés afin de générer des échantillons d'origine correspondants ;
b. préparer une bibliothèque pour chaque sujet testé dans laquelle les fragments d'acide nucléique sont codés par un code à barres et dans laquelle chaque bibliothèque correspond à un échantillon d'origine spécifique ;
c. ajouter un premier nombre d'unités d'acide nucléique de chaque échantillon d'origine pour former un premier échantillon de test groupé ;
d. effectuer une sélection de la taille des fragments sur le premier échantillon de test groupé et isoler la population d'acides nucléiques cibles en suspension pour former un deuxième échantillon de test groupé enrichi pour ladite population d'acides nucléiques cibles ;
e. déterminer l'abondance d'une population d'acides nucléiques cibles dans chaque échantillon d'origine ;
f. calculer une valeur de décalage numérique unique pour chaque échantillon d'origine ;
g. ajouter un deuxième nombre d'unités d'acide nucléique de chaque échantillon d'origine sur la base de la valeur de décalage numérique unique pour former un troisième échantillon de test groupé enrichi pour ladite population d'acide nucléique cible ;
h. effectuer une seconde sélection de taille de fragment sur le troisième échantillon de test groupé et isoler la population d'acides nucléiques cibles en suspension pour former un quatrième échantillon de test groupé enrichi pour ladite population d'acides nucléiques cibles et comprenant des proportions sensiblement égales de chaque échantillon d'origine, dans lequel ledit quatrième échantillon de test groupé est prêt pour un test diagnostique.

8. Procédé selon la revendication 7, dans lequel l'étape f est réalisée par séquençage ; dans lequel ledit séquençage est un séquençage à extrémités appariées ;
dans lequel l'étape f est réalisée par PCR quantitative ; ou
dans lequel l'étape f est réalisée par PCR numérique, éventuellement dans lequel ladite PCR numérique est une PCR numérique à gouttelettes.

9. Procédé selon la revendication 7, comprenant en outre l'étape consistant à séquencer ledit quatrième échantillon de test groupé et à dépister la population d'acides nucléiques cibles pour y chercher des anomalies génétiques ; ou comprenant en outre l'étape consistant à apparier les fragments d'acide nucléique dans le quatrième échantillon de test groupé avec l'échantillon d'origine respectif.

10. Procédé selon la revendication 7, dans lequel ledit acide nucléique est de l'ADN génomique ;
dans lequel ledit acide nucléique est de l'ADN FFPE ;
dans lequel ledit acide nucléique est de l'ARN ;
dans lequel ledit acide nucléique est de l'ADN extracellulaire ; ou
dans lequel ledit acide nucléique est isolé à partir de sang total.

11. Procédé selon la revendication 7, dans lequel ladite valeur de décalage numérique unique est calculée en divisant l'abondance de la population d'acides nucléiques cibles déterminée à l'étape e par le premier nombre d'unités d'acides nucléiques.

12. Procédé selon la revendication 7, dans lequel ladite population d'acides nucléiques cibles est la fraction fœtale dudit ADN extracellulaire ;
dans lequel ladite population d'acides nucléiques cibles est la fraction tumorale dudit ADN extracellulaire ;
dans lequel ladite population d'acides nucléiques cibles sont des fragments comprenant une signature de méthylation particulière ;
dans lequel ladite signature de méthylation est une hyperméthylation ou une hypométhylation ;
dans lequel ladite population d'acides nucléiques cibles est enrichie en fragments d'une longueur comprise dans une plage prédéterminée ;
dans lequel ladite population d'acides nucléiques cibles est enrichie en fragments d'une longueur prédéterminée ;
dans lequel ladite population d'acides nucléiques cibles est enrichie en fragments comprenant une signature de méthylation particulière ;
dans lequel ladite signature de méthylation est une hyperméthylation ou une hypométhylation ;
dans laquelle ladite sélection de la taille des fragments est effectuée par électrophorèse sur gel ; ou
dans laquelle lesdits premier et deuxième nombres d'unités d'acide nucléique sont sélectionnées dans le groupe constitué par les microlitres, les nanogrammes et les moles.

13. Procédé selon la revendication 7, comprenant en outre l'étape consistant à effectuer le séquençage du génome entier.

14. Procédé selon la revendication 7, dans laquelle ledit échantillon de test regroupé comprend entre 2 et 1000 échantillons différents.

15. Procédé pour améliorer la sensibilité et la résolution des tests de diagnostic génétique comprenant les étapes consistant à :
a. isoler et purifier l'acide nucléique d'au moins un sujet testé afin de générer au moins un échantillon d'origine ;
b. préparer une bibliothèque d'acides nucléiques pour ledit au moins un sujet testé dans laquelle les fragments d'acide nucléique sont codés par un code à barres et dans laquelle ladite bibliothèque d'acides nucléiques correspond audit au moins un échantillon d'origine ;
c. ajouter un premier nombre d'unités d'acide nucléique de ladite bibliothèque d'acides nucléiques pour former un premier échantillon de test ;
d. déterminer la distribution de la taille des fragments dans ladite bibliothèque d'acides nucléiques ;
e. calculer l'abondance d'une population d'acides nucléiques cibles dans ladite bibliothèque d'acides nucléiques ;
f. calculer une valeur de décalage numérique unique pour ladite bibliothèque d'acides nucléiques ;
g. ajouter un deuxième nombre d'unités d'acide nucléique de ladite bibliothèque d'acides nucléiques sur la base de la valeur de décalage numérique unique pour former un deuxième échantillon de test ; et
h. effectuer une sélection de la taille des fragments sur le deuxième échantillon de test et isoler la population d'acides nucléiques cibles en suspension pour former un troisième échantillon de test enrichi pour ladite population d'acides nucléiques cibles, dans lequel ledit troisième échantillon de test est prêt pour un test diagnostique.
